# EUROPÄISCHE PATENTANMELDUNG

(11) **EP 0 693 491 A1**
(43) Veröffentlichungstag der Anmeldung: **24.01.1996**
(21) Anmeldenummer: 95110629.3
(22) Anmeldetag: 07.07.1995
(51) Int. Cl.: C07D 413/04, A61K 31/42, A61K 31/425, C07D 263/20, C07D 413/14, C07D 417/04, C07D 417/14

(54) **5-Gliedrige-heteroaryl-oxazolidinone als antibakterielle Arzneimittel**

(30) Priorität: 20.07.1994 DE 19944425613
(71) Anmelder: BAYER AG, D-51368 Leverkusen (DE)
(72) Erfinder: Riedl, Bernd, Dr., D-42115 Wuppertal (DE); Häbich, Dieter, Dr., D-42115 Wuppertal (DE); Stolle, Andreas, Dr., D-42115 Wuppertal (DE); Wild, Hanno, Dr., Orange, Connecticut 06477 (US); Endermann, Rainer, Dr., D-42113 Wuppertal (DE); Bremm, Klaus Dieter, Dr., D-45661 Recklinghausen (DE); Kroll, Hein-Peter, Dr., D-42115 Wuppertal (DE); Labischinski, Harald, Prof. Dr., D-42109 Wuppertal (DE); Schaller, Klaus, Dr., D-42109 Wuppertal (DE); Werling, Hans-Otto, Dr., D-42113 Wuppertal (DE)

(57) **Zusammenfassung**

Die vorliegende Erfindung betrifft 5-gliedrige Heteroaryl-oxazolidinone, der allgemeinen Formel (I) 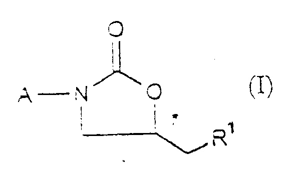 in welcher
R¹
für Azido, Hydroxy oder für eine Gruppe der Formel -OR², -O-SO₂R³ oder -NR⁴R⁵ steht, worin
R²
geradkettiges oder verzweigtes Acyl mit bis zu 8 Kohlenstoffatomen oder eine Hydroxyschutzgruppe bedeutet,
R³
geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
R⁴ und R⁵
gleich oder verschieden sind und Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten,
oder
R⁴ oder R⁵
eine Gruppe der Formel -CO-R⁶ bedeutet,
worin
R⁶
Cycloalkyl mit 3 bis 6 Kohlenstoffatomen, geradkettiges oder verzweigtes Alkyl mit bis zu 8 Kohlenstoffatomen, Phenyl oder Wasserstoff bedeutet,
A
für einen über ein Kohlenstoffatom direkt gebundenen 5-gliedrigen aromatischen Heterocyclus mit bis zu 3 Heteroatomen aus der Reihe S, N und/oder O steht, der zusätzlich einen annellierten Benzol-oder Naphthylring besitzen kann.

Verfahren zu ihrer Herstellung und ihre Verwendung als Arzneimittel, insbesondere als antibakterielle Arzneimittel.

## Beschreibung

Die vorliegende Erfindung betrifft 5-gliedrige Heteroaryl-oxazolidinone, Verfahren zu ihrer Herstellung und ihre Verwendung als Arzneimittel, insbesondere als antibakterielle Arzneimittel.

Aus den Publikationen US 5 254 577, US 4 705 799, EP 311 090, US 4 801 600, US 4 921 869, US 4 965 268, EP 312 000 und C.H. Park et al., J. Med. Chem. 35, 1156 (1992) sind N-Aryloxazolidinone mit antibakterieller Wirkung bekannt.

Aus der Publikation EP 300 272 sind Oxazolidinone mit Wirkungen auf das Zentralnervensystem bekannt.

Die vorliegende Erfindung betrifft 5-gliedrige Heteroaryl-oxazolidinone der allgemeinen Formel (I) 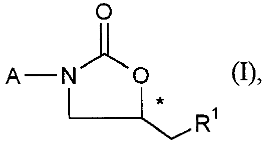 in welcher
- R¹: für Azido, Hydroxy oder für eine Gruppe der Formel - OR², -O-SO₂R³ oder -NR⁴R⁵ steht,
worin
- R²: geradkettiges oder verzweigtes Acyl mit bis zu 8 Kohlenstoffatomen oder eine Hydroxyschutzgruppe bedeutet,
- R³: geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
- R⁴ und R⁵: gleich oder verschieden sind und Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten,
oder
- R⁴ oder R⁵: eine Gruppe der Formel -CO-R6 bedeutet, worin
- R⁶: Cycloalkyl mit 3 bis 6 Kohlenstoffatomen, geradkettiges oder verzweigtes Alkyl mit bis zu 8 Kohlenstoffatomen , Phenyl oder Wasserstoff bedeutet,
- A: für einen über ein Kohlenstoffatom direkt gebundenen 5-gliedrigen aromatischen Heterocyclus mit bis zu 3 Heteroatomen aus der Reihe S, N und/oder O steht, der zusätzlich einen annellierten Benzol-oder Naphthylring besitzen kann, wobei die Cyclen gegebenenfalls jeweils bis zu 3-fach gleich oder verschieden durch Carboxy, Halogen, Cyano, Mercapto, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel - NR⁷R⁸ substituiert sein kann,
worin
- R⁷ und R⁸: gleich oder verschieden sind und Wasserstoff, geradkettiges oder verzweigtes Alkyl mit bis zu 4 Kohlenstoffatomen oder Phenyl bedeuten, oder gemeinsam mit dem Stickstoffatom einen 5-bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,
und/oder
die Cyclen gegebenenfalls durch eine Gruppe der Formel - NR^{7'}R^{8'} substituiert sind,
worin
- R^{7'} und R^{8'}: gleich oder verschieden sind und die oben angegebene Bedeutung von R⁷ und R⁸ haben und mit diesen gleich oder verschieden sind,
und/oder
die Cyclen gegebenenfalls durch (C₂-C₈)-Alkenylphenyl, Phenyl oder durch einen 5- oder 6-gliedrigen gesättigten oder ungesättigten Heterocyclus mit bis zu 3 Heteroatomen aus der Reihe S, N und/oder O substituiert sind, die ihrerseits gegebenenfalls durch eine Gruppe der Formel - CO-NR⁹R¹⁰, NR¹¹R¹², -NR¹³-S(O)₂-R¹⁴, R¹⁵R¹⁶N-SO₂- oder R¹⁷-S(O)ₐ- substituiert sind,
worin
- a: eine Zahl 0, 1 oder 2 bedeutet,
- R⁹, R¹⁰, R¹³, R¹⁵ und R¹⁶: gleich oder verschieden sind und Wasserstoff, geradkettiges oder verzweigtes Alkyl mit bis zu 6 Kohlenstoffatomen oder Phenyl bedeuten,
- R¹¹ und R¹²: gleich oder verschieden sind und die oben angegebene Bedeutung von R⁷ und R⁸ haben und mit diesen gleich oder verschieden sind,
- R¹⁴ und R¹⁷: gleich oder verschieden sind und die oben angegebene Bedeutung von R³ haben und mit dieser gleich oder verschieden sind,
und/oder ihrerseits gegebenenfalls bis zu 2-fach gleich oder verschieden durch Carboxy, Halogen, Cyano, Mercapto, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR¹⁸R¹⁹ substituiert sein kann,
worin
- R¹⁸ und R¹⁹: die oben angegebene Bedeutung von R⁷ und R⁸ haben und mit diesen gleich oder verschieden sind,
und/oder
die Cyclen gegebenenfalls durch einen Rest der Formel 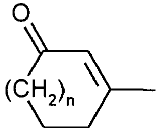 substituiert sind
worin
- n: eine Zahl 0, 1 oder 2 bedeutet
und deren Salze und S-Oxide.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomerer einheitlichen Bestandteile trennen.

Physiologisch unbedenkliche Salze der 5-gliedrigten Heteroaryl-oxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium-oder Kaliumsalze), Erdalkalisalze (z. B. Calcium-oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Heterocyclus unter dem Substituenten A im Fall der direkten Anbindung an das Oxazolidinongerüst steht im Rahmen der Erfindung für einen 5-gliedrigen aromatischen Ring, der als Heteroatome bis zu 3 Sauerstoff-, Schwefel- und/oder Stickstoffatome enthalten und zusätzlich einen annellierten Benzol- oder Naphthylring besitzen kann. Beispielsweise werden genannt: Pyrrolyl, Imidazolyl, Furyl, Thienyl, Thiazolyl, Oxazolyl, Isothiazolyl, Isoxazolyl, Furazanyl, Indolyl, Benzo[b]thienyl, Naphtho[2,3-b]thienyl, Benzo[b]thiazolyl, Benzo[b]furanyl oder Benzo[b]imidazolyl. Bevorzugt sind Pyrrolyl, Imidazolyl, Furyl, Thienyl, Isothiazolyl, Thiazolyl, Isoxazolyl, Furazanyl, Oxazolyl, Benzo[b]thienyl, Benzo[b]imidazolyl und Benzo[b]thiazolyl.

Im weiteren Substitutionsfeld steht Heteroyclus auch für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Dazu gehören auch über N-gebundene, 5- bis 6-gliedrige gesättigte Heterocyclen, die außerdem als Heteroatome bis zu 2 Sauerstoff-, Schwefel- und/oder Stickstoffatome enthalten können, wie beispielsweise Piperidyl, Morpholinyl oder Piperazinyl oder Pyrrolidinyl. Besonders bevorzugt sind Piperidyl und Pyrrolidinyl.

Hydroxyschutzgruppe im Rahmen der oben angegebenen Definition steht im allgemeinen für eine Schutzgruppe aus der Reihe: Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl oder Tetrahydropyranyl.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Bevorzugt sind Verbindungen der allgemeinen Formel (I),
in welcher
- R¹: für Azido, Hydroxy oder für eine Gruppe der Formel - OR², -OSO₂R³ oder -NR⁴R⁵ steht,
worin
- R²: geradkettiges oder verzweigtes Acyl mit bis zu 6 Kohlenstoffatomen oder Benzyl bedeutet,
- R³: geradkettiges oder verzweigtes Alkyl mit bis zu 3 Kohlenstoffatomen, Phenyl oder Toluolyl bedeutet,
- R⁴ und R⁵: gleich oder verschieden sind und Cyclopropyl, Cycylopentyl, Cyclohexyl, Wasserstoff, Phenyl oder geradkettiges oder verzweigtes Alkyl mit bis zu 6 Kohlenstoffatomen, tert. Butoxycarbonyl oder Benzyloxycarbonyl bedeuten,
oder
- R⁴ oder R⁵: eine Gruppe der Formel -CO-R⁶ bedeutet,
worin
- R⁶: Cyclopropyl, Cyclobutyl, Cyclopentyl, Cyclohexyl oder geradkettiges oder verzweigtes Alkyl mit bis zu 6 Kohlenstoffatomen, Phenyl oder Wasserstoff bedeutet,
- A: für über ein Kohlenstoffatom direkt gebundenes Pyrrolyl, Imidazolyl, Furyl, Thienyl, Thiazolyl, Oxazolyl, Isothiazolyl, Isoxazolyl oder Furazanyl steht, oder für ebenfalls über ein Kohlenstoffatom des 5-gliedrigen Ringes direkt gebundenes Indolyl, Benzo[b]thienyl, Naphto[2,3-b]thienyl, Benzo[b]thiazolyl, Benzo[b]imidazolyl oder Benzo[b]furanyl steht, wobei die Cyclen gegebenenfalls jeweils bis zu 3-fach gleich oder verschieden durch Carboxy, Fluor, Chlor, Brom, Jod, Cyano, Mercapto, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel - NR⁷R⁸ gegebenenfalls substituiert sein kann,
worin
- R⁷ und R⁸: gleich oder verschieden sind und Wasserstoff, geradkettiges oder verzweigtes Alkyl mit bis zu 3 Kohlenstoffatomen oder Phenyl bedeuten, oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind, und/oder die Cyclen gegebenenfalls durch eine Gruppe der Formel - NR^{7'}R^{8'} substituiert sind,
worin
- R^{7'} und R^{8'}: die oben angegebene Bedeutung von R⁷ und R⁸ haben und mit diesen gleich oder verschieden sind,
und/oder
die Cyclen gegebenenfalls durch (C₂-C₄)-Alkenylphenyl, Phenyl, Pyridyl oder Thienyl substituiert sind, die ihrerseits gegebenenfalls durch eine Gruppe der Formel -CO-NR⁹R¹⁰, -NR¹¹R¹², -NR¹³-SO₂-R¹⁴, R¹⁵R¹⁶N-SO₂- oder -R¹⁷-S(O)ₐ- substituiert sind,
worin
- a: eine Zahl 0, 1 oder 2 bedeutet,
- R⁹, R¹⁰, R¹³, R¹⁵ und R¹⁶: gleich oder verschieden sind und Wasserstoff, geradkettiges oder verzweigtes Alkyl mit bis zu 4 Kohlenstoffatomen oder Phenyl bedeuten,
- R¹¹ und R¹²: gleich oder verschieden sind und die oben angegebene Bedeutung von R⁷ und R⁸ haben und mit diesen gleich oder verschieden sind,
- R¹⁴ und R¹⁷: gleich oder verschieden sind und die oben angegebene Bedeutung von R³ haben und mit dieser gleich oder verschieden sind,
und/oder ihrerseits gegebenenfalls bis zu 2-fach gleich oder verschieden durch Carboxy, Fluor, Chlor, Brom, Jod, Cyano, Mercapto, Trifluormethyl, Formyl, Nitro, Phenyl geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR¹⁸R¹⁹ gegebenenfalls substituiert sein kann,
worin
- R¹⁸ und R¹⁹: die oben angegebene Bedeutung von R⁷ und R⁸ haben und mit diesen gleich oder verschieden sind,
und/oder
die Cyclen gegebenenfalls durch einen Rest der Formel 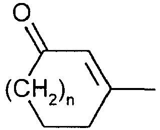 substituiert sind,
worin
- n: eine Zahl 0, 1 oder 2 bedeutet
und deren Salze und S-Oxide.

Besonders bevorzugt sind Verbindungen der allgemeinen Formel (I), in welcher
- R¹: für Azido, Hydroxy oder für eine Gruppe der Formel - OR², -OSO₂R³ oder -NR⁴R⁵ steht, worin
- R²: geradkettiges oder verzweigtes Acyl mit bis zu 6 Kohlenstoffatomen bedeutet,
- R³: Methyl, Ethyl, Phenyl oder Toluolyl bedeutet,
- R⁴ und R⁵: gleich oder verschieden sind und Cyclopropyl, Cyclopentyl, Cyclohexyl, Wasserstoff, Phenyl oder geradkettiges oder verzweigtes Alkyl mit bis zu 5 Kohlenstoffatomen bedeuten,
oder
- R⁴ oder R⁵: eine Gruppe der Formel -CO-R⁶ bedeutet,
worin
- R⁶: Cyclopropyl, Cyclopentyl, Cyclohexyl oder geradkettiges oder verzweigtes Alkyl mit bis zu 4 Kohlenstoffatomen, Wasserstoff oder Phenyl bedeutet,
- A: für über ein Kohlenstoffatom direkt gebundenes Pyrrolyl, Imidazolyl, Furyl, Thienyl, Thiazolyl, Isothiazolyl, Isoxazolyl, Furazanyl oder Oxazolyl steht, oder für ebenfalls über ein Kohlenstoffatom des 5-gliedrigen Ringes direkt gebundenes Indolyl, Benzo[b]thienyl, Benzo[b]imidazolyl, Benzo[b]furanyl oder Benzo[b]thiazolyl steht, wobei die Cyclen gegebenenfalls jeweils bis zu 2-fach gleich oder verschieden durch Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR⁷R⁸ gegebenenfalls substituiert sein kann,
worin
- R⁷ und R⁸: gleich oder verschieden sind und Wasserstoff oder Methyl bedeuten,
oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind,
und/oder gegebenenfalls durch eine Gruppe der Formel - NR^{7'}R^{8'} substituiert sind,
worin
- R^{7'} und R^{8'}: die oben angegebene Bedeutung von R⁷ und R⁸ haben und mit diesen gleich oder verschieden sind,
und/oder
die Cyclen gegebenfalls durch 2-Phenylvinyl, Phenyl, Pyridyl oder Thienyl substituiert sind, die ihrerseits gegebenenfalls durch eine Gruppe der Formel -CO-NR⁹R¹⁰ oder -NR¹¹R¹² substituiert sind,
worin
- R⁹ und R¹⁰: gleich oder verschieden sind und Wasserstoff oder Methyl bedeuten,
- R¹¹ und R¹²: gleich oder verschieden sind und die oben angegebene Bedeutung von R⁷ und R⁸ haben und mit diesen gleich oder verschieden sind,
und/oder ihrerseits gegebenenfalls bis zu 2-fach gleich oder verschieden durch Carboxy, Fluor, Chlor, Brom, Jod, Cyano, Formyl, Trifluormethyl, Nitro, Phenyl, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR¹⁸R¹⁹ gegebenenfalls substituiert sein kann,
worin
- R¹⁸ und R¹⁹: die oben angegebene Bedeutung von R⁷ und R⁸ haben und mit diesen gleich oder verschieden sind,
und/oder
die Cyclen gegebenenfalls durch einen Rest der Formel 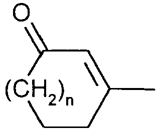 substituiert sind,
worin
- n: eine Zahl 0, 1 oder 2 bedeutet
und deren Salze und S-Oxide.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formeln (II) oder (III)

   A-N=C=O (II)

   oder

   A-CO-N₃ (III),

   in welchen
   - A: die oben angegebene Bedeutungen hat,
   mit Lithiumbromid/(C₄H₉)₃ P(O) und Epoxiden der allgemeinen Formel (IV) 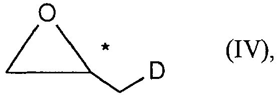 in welcher
   - D: für C₁-C₆-Acyloxy steht,
   in inerten Lösemitteln, gegebenenfalls in Anwesenheit einer Base,
   umsetzt,
   und im Fall R¹ = OH durch eine typische Esterverseifung oder durch eine typische Umesterung die Hydroxyfunktion freisetzt,
   oder
[B] Verbindungen der allgemeinen Formel (V)

   A-NH-CO₂-L (V),

   in welcher
   - A: die oben angegebene Bedeutung hat
   und
   - L: für eine typische Schutzgruppe, vorzugsweise Benzyl, steht,
   in inerten Lösemitteln und in Anwesenheit einer Base, beispielsweise Lithiumalkylen oder Lithium-N-alkyl- oder Lithium-N-silylalkylamiden, vorzugsweise N-Butyllithium, mit Epoxiden der allgemeinen Formel (IV) umsetzt, oder
[C] im Fall R¹ = OH, zunächst Verbindungen der allgemeinen Formel (III) durch Abspaltung von Stickstoff in Alkoholen in die Verbindungen der allgemeinen Formel (Va)

   A-NH-CO₂-T (Va),

   in welcher
   - A: die oben angegebene Bedeutung hat
   und
   - T: für geradkettiges oder verzweigtes C₂-C₆-Alkyl, vorzugsweise n-Butyl, steht,
   überführt,
   und in einem zweiten Schritt wie unter [A] beschrieben in inerten Lösemitteln und in Anwesenheit einer Base, vorzugsweise Lithium-N-alkyl- oder N-Silylalkylamiden oder n-Butyllithium und Epoxiden der allgemeinen Formel (IV) umsetzt,
   oder
[D] Verbindungen der allgemeinen Formel (VI) 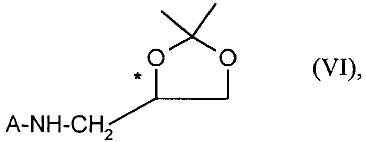 in welcher
   - A: die oben angegebene Bedeutung hat,
   entweder direkt mit Säuren und Kohlensäurediethylester
   umsetzt,
   oder zunächst durch Umsetzung der Verbindungen der allgemeinen Formel (VI) mit Säuren die Verbindungen der allgemeinen Formel (VII) 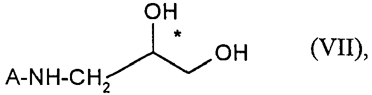 in welcher
   - A: die oben angegebene Bedeutung hat,
   herstellt,
   und anschließend in Anwesenheit eines Hilfsmittels in inerten Lösemitteln cyclisiert, oder
[E] zunächst Verbindungen der allgemeinen Formel (la) 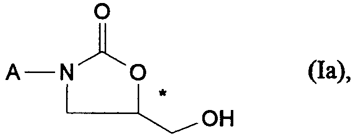 in welcher
   - A: die oben angegebene Bedeutung hat,
   durch Umsetzung mit (C₁-C₄)-Alkyl- oder Phenylsulfonsäurechloriden in inerten Lösemitteln und in Anwesenheit einer Base in die entsprechenden Verbindungen der allgemeinen Formel (Ib) 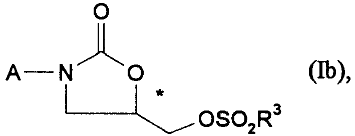 in welcher
   - A und R³: die oben angegebene Bedeutung haben,
   überführt,
   anschließend mit Natriumazid in inerten Lösemitteln die Azide der allgemeinen Formel (Ic) 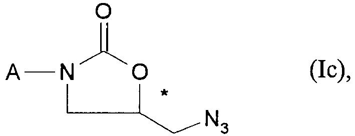 in welcher
   - A: die oben angegebene Bedeutung hat,
   herstellt,
   in einem weiteren Schritt durch Umsetzung mit (C₁C₄-O)₃-P oder PPh₃, vorzugsweise (CH₃O)₃P in inerten Lösemitteln und mit Säuren in die Amine der allgemeinen Formel (Id) 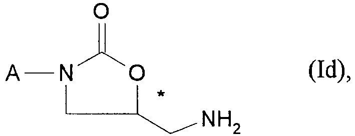 in welcher
   - A: die oben angegebene Bedeutung hat,
   überführt,
   und durch Umsetzung mit Acetanhydrid oder anderen Acylierungsmitteln der allgemeinen Formel (VIII)

   R²⁰-CO-R⁶ (VIII)

   in welcher
   - R⁶: die oben angegebene Bedeutung hat,
   und
   - R²⁰: für Halogen, vorzugsweise für Chlor, oder für den Rest -O-CO-R⁶ steht,
   in inerten Lösemitteln die Verbindungen der allgemeinen Formel (Ie) 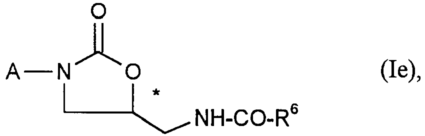 in welcher
   - A und R⁶: die oben angegebene Bedeutung haben,
   herstellt
   oder
[F] Verbindungen der allgemeinen Formel (Ie) durch eine Halogenierung, gegebenenfalls in Anwesenheit eines Silberkatalysators, in die Verbindungen der allgemeinen Formel (If) 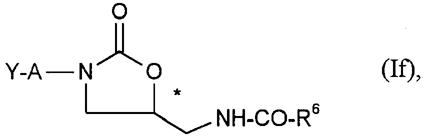 in welcher
   - Y: für Halogen, vorzugsweise für Brom oder Jod steht,
   und
   - A und R⁶: die oben angegebene Bedeutung haben,
   überführt,
   oder
[G] Verbindungen der allgemeinen Formel (If) mit Verbindungen der allgemeinen Formel (IX)

   A'-R²¹ (IX),

   in welcher
   - A': für einen der unter A oben aufgeführten, gegebenenfalls substituierten, monocyclischen Heterocyclen, Phenyl oder (C₂-C₈)-Alkenylphenyl steht,
   und
   - R²¹: für den Boronsäurerest -B(OH)₂ oder
   für einen zinnorganischen Rest der Formel -SnR²²R²³R²⁴ steht,
   worin
   - R²², R²³ und R²⁴: gleich oder verschieden sind und C₁-C₄-Alkyl bedeuten,
   in inerten Lösemitteln und in Anwesenheit eines Palladiumkatalysators umsetzt,
   und im Fall der S-Oxide eine Oxidation durchführt,
   und im Fall R⁴, R⁵, R⁹, R¹⁰, R¹¹, R¹², R¹³, R¹⁵, R¹⁶, R¹⁸ und R¹⁹ ≠ H eine Alkylierung nach üblichen Methoden durchführt,
   und gegebenenfalls weitere Substituenten oder bereits vorhandene funktionelle Gruppen nach üblichen Methoden, wie beispielsweise Redoxreaktionen, Substitutionsreaktionen und/oder Verseifungen oder Ein- und Abbau von Schutzgruppen, einführt bzw. derivatisiert.

Die erfindungsgemäßen Verfahren können durch folgende Formelschemata beispielhaft erläutert werden: 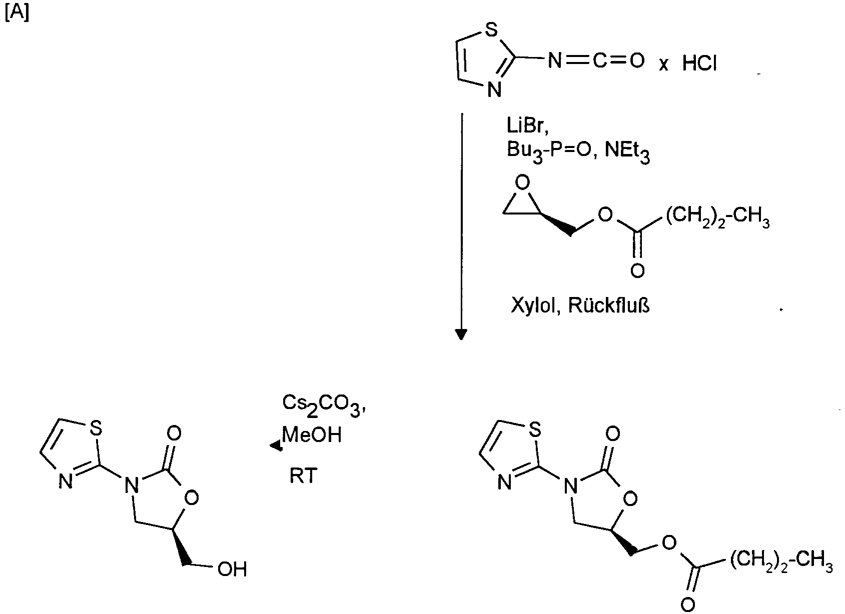 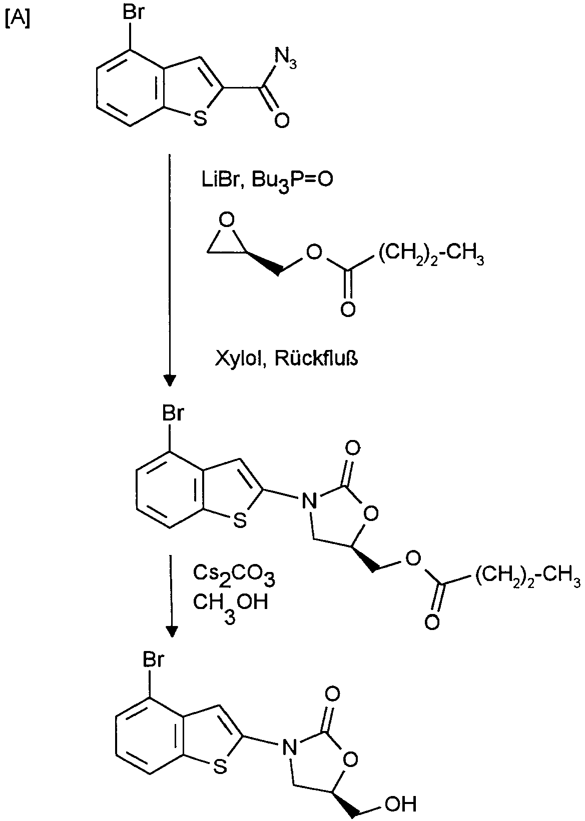 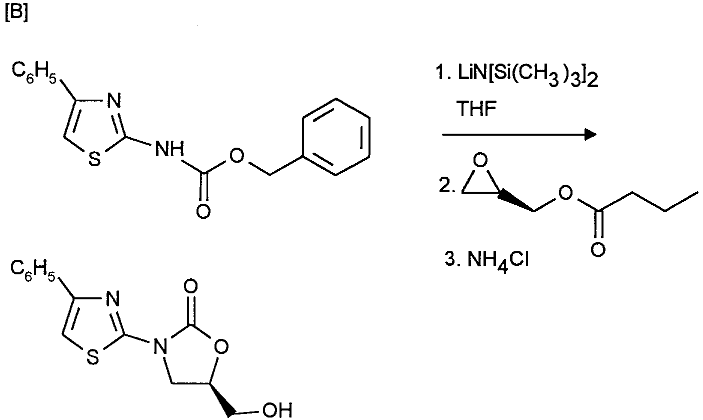 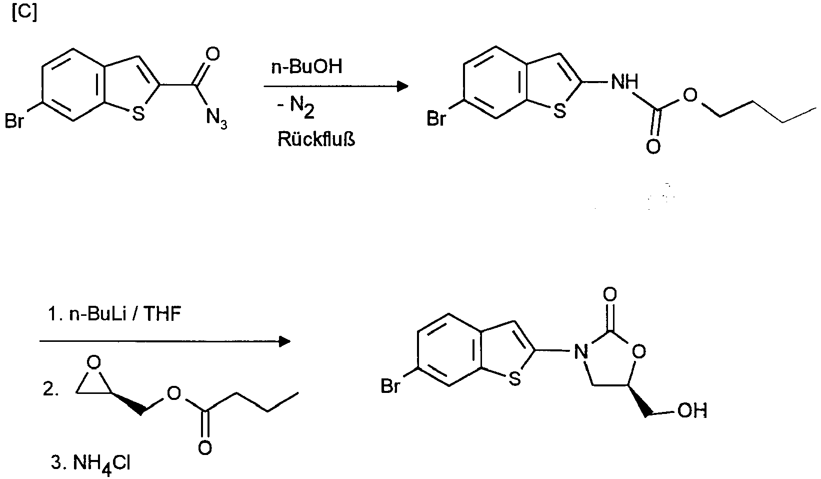 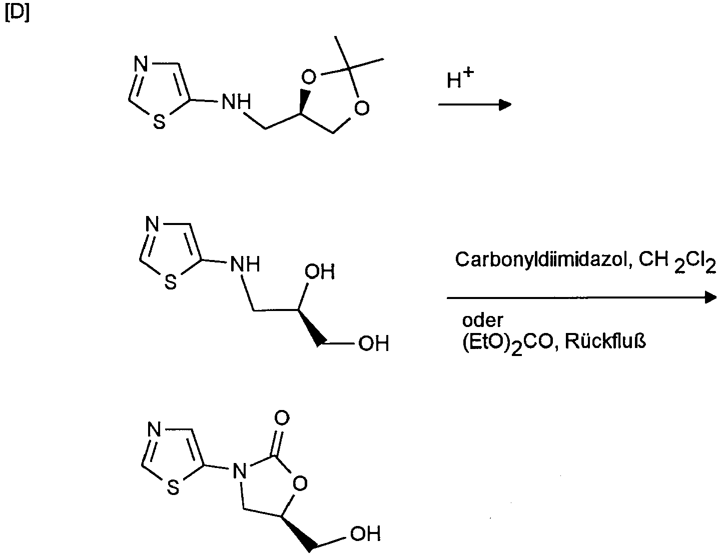 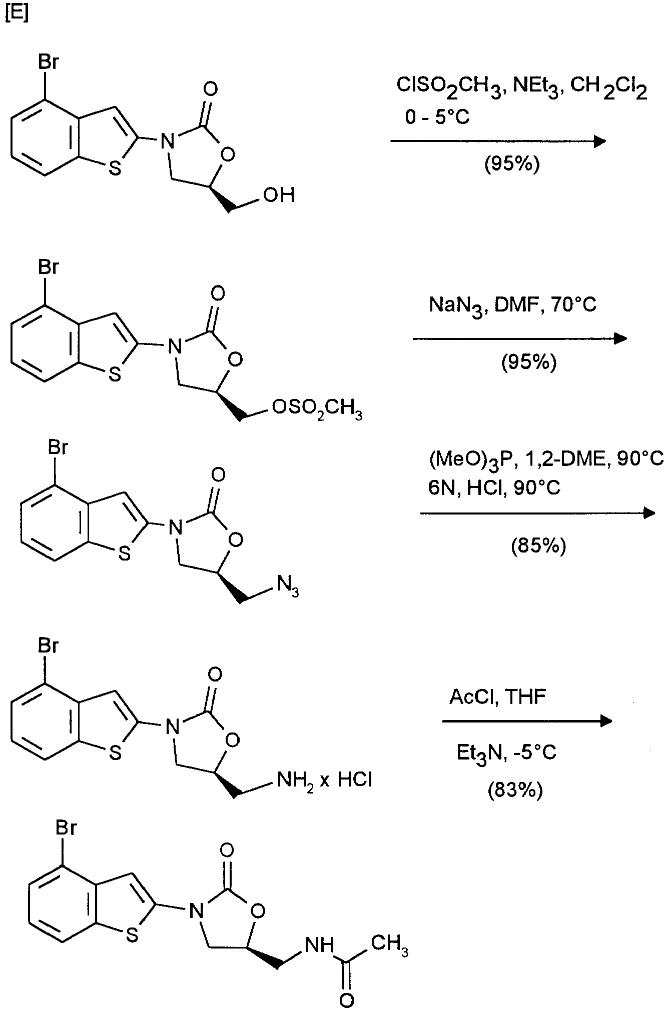 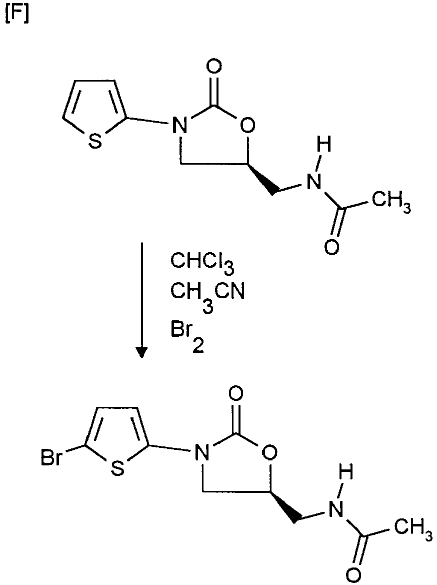 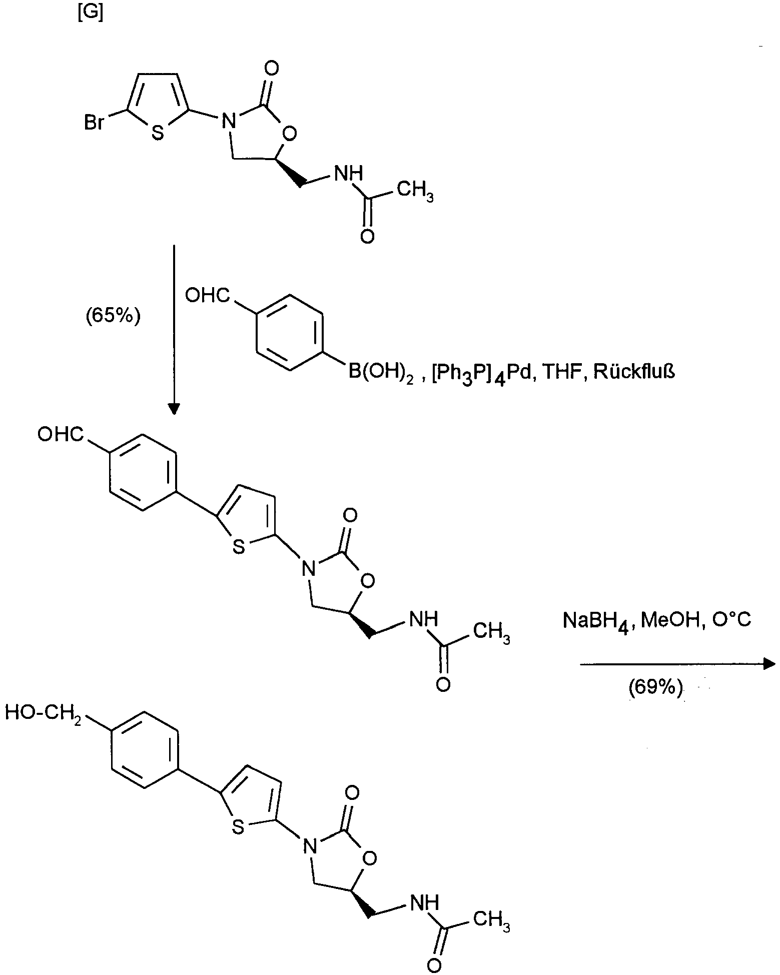 Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenysilylamid oder Lithiumalkyle wie n-Butyllithium.

Die Base wird in einer Menge von 1 mol bis 10 mol, bevorzugt von 1 mol bis 3 mol, bezogen auf 1 mol der Verbindungen der allgemeinen Formeln (II), (III), (IV) und (Va), eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Das Verfahren [A] erfolgt bevorzugt in Xylol oder Dichlorbenzol, gegebenenfalls in Anwesenheit von Triethylamin, unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur, durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [B] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran mit Lithium-bis-trimethylsilylamid oder n-Butyllithium, in einem Temperaturbereich von -100°C bis +20°C, vorzugsweise von -75°C bis -40°C.

Für das Verfahren [C] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylalkylverbindungen oder n-Butyllithium. Besonders bevorzugte ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Die Cyclisierung [D] wird in Anwesenheit eines Hilfsmittels und/oder Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (VI), eingesetzt.

Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester oder Chlorameisensäuretrichlormethylester.

Als Lösemittel eignen sich die oben aufgeführten Halogenkohlenwasserstoffe. Bevorzugt ist Methylenchlorid.

Die Cyclisierungen erfolgen im allgemeinen in einem Temperaturbereich von -20°C bis 100°C, vorzugsweise bei - 20°C bis Raumtemperatur.

Die Acylierung [E] erfolgt im allgemeinen in einem der oben aufgeführten Ether oder Halogenkohlenwasserstoffen, vorzugsweise Tetrahydrofuran oder Methylenchlorid, in einem Temperaturbereich von -30°C bis 50°C, bevorzugt von-10°C bis Raumtemperatur.

Die Kupplungsreaktionen [G] mit den Boronsäure- und Zinnarylverbindungen erfolgt ebenfalls in einem der oben aufgeführten Ether oder Kohlenwasserstoffe, vorzugsweise Tetrahydrofuran oder Toluol und in Anwesenheit eines Palladiumkomplexes.

Als Palladiumkomplexe eignen sich beispielsweise Pd[P(C₆H₅)₃]₄, [(C₆H₅)₃P]₂PdCl₂ oder (C₆H₅CN)₂PdCl₂. Bevorzugt ist [(C₆H₅)₃P]₄Pd.

Die Umsetzung erfolgt in einem Temperaturbereich von Raumtemperatur bis 150°C, vorzugsweise bei der Siedetemperatur des jeweiligen Lösemittels.

Die Reduktionen erfolgen im allgemeinen mit Hydriden in inerten Lösemitteln oder mit Boranen, Diboranen oder ihren Komplexverbindungen.

Bevorzugt werden die Reduktionen mit Hydriden, wie komplexen Borhydriden oder Aluminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxyethoxy)aluminiumhydrid oder Boran-Tetrahydrofuran eingesetzt.

Die Reduktion erfolgt im allgemeinen in einem Temperaturbereich von - 50°C bis zum jeweiligen Siedepunkt des Lösemittels, bevorzugt von -20°C bis +90°C.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Oxidation zum S-oxid erfolgt im allgemeinen in einem der oben aufgeführten Lösemittel, vorzugsweise in Methylenchlorid mit Oxidationsmitteln wie beispielsweise Metachlorperbenzoesäure, Wasserstoffperoxid oder Peressigsäure, vorzugsweise mit Metachlorperbenzoesäure in einem Temperaturbereich von 0°C bis 80°C, bevorzugt von 20°C bis 60°C.

Die Abspaltung der Hydroxyschutzgruppen erfolgt im allgemeinen nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Abspaltung der Aminoschutzgruppe erfolgt im allgemeinen ebenfalls nach üblichen Methoden und zwar vorzugsweise Boc mit Salzsäure in Dioxan, Fmoc mit Piperidin und Z mit HBr/HOAc oder durch Hydrogenolyse.

Die oben aufgeführten anderen Derivatisierungsreaktionen erfolgen im allgemeinen nach denen in Compendium of Organic Synthetic Methods, T.T. Harrison und S. Harrison, Wiley Interscience, publizierten Methoden.

Bevorzugt werden Redoxreaktionen, reduktive Aminierung, Umesterung und die Halogenisierung von Methylgruppen mit N-Bromsuccinimid (NBS) oder N-Chlorsuccinimid (NCS) aufgeführt, die im folgenden beispielhaft erläutert werden.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Dichlormethan, Dimethylsulfoxid und Dimethylformamid.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Amidierung und die Sulfoamidierung erfolgen im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan oder Trichlorethylen, Kohlenwasserstoffe wie Benzol, Xylol, Toluol, Hexan, Cyclohexan, oder Erdölfraktionen, Nitromethan, Dimethylformamid, Acetonitril oder Tetrahydrofuran. Ebenso ist es möglich, Gemische der Lösemittel einzusetzen. Besonders bevorzugt sind Dichlormethan und Tetrahydrofuran.

Als Basen für die Amidierung und die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung und die Sulfoamidierung werden im allgemeinen in einem Temperaturbereich von 0°C bis 150°C, bevorzugt bei 25°C bis 40°C, durchgeführt.

Die Amidierung und die Sulfoamidierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung und der Sulfoamidierung wird die Base im allgemeinen in einer Menge von 1 bis 3 Mol, bevorzugt von 1 bis 1,5 Mol, bezogen auf 1 Mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphoniumhexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder 4-Dimethylaminopyridin.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol, bezogen auf 1 Mol des Esters, eingesetzt. Besonders bevorzugt verwendet man molare Mengen der Reaktanden.

Die Veresterung erfolgt im allgemeinen mit den entsprechenden Alkoholen in Anwesenheit von Säuren, vorzugsweise Schwefelsäure, in einem Temperaturbereich von 0°C bis 150°C, vorzugsweise von 50°C bis 100°C, und Normaldruck.

Die Verbindungen der allgemeinen Formeln (IV), (VIII) und (IX) sind bekannt oder können nach üblichen Methoden hergestellt werden.

Die Verbindungen der allgemeinen Formel (VII) sind größtenteils neu und können beispielsweise wie oben beschrieben hergestellt werden.

Die Verbindungen der allgemeinen Formel (II) sind teilweise bekannt oder neu und können dann beispielsweise hergestellt werden, indem man die entsprechenden Amine mit Chlorameisensäuretrichlorethylester in einem der oben aufgeführten Lösemittel, vorzugsweise Xylol bei Rückflußtemperatur umsetzt.

Die Verbindungen der allgemeinen Formel (III) sind teilweise bekannt oder neu und können dann beispielsweise hergestellt werden, indem man ausgehend von den entsprechenden Carbonsäuren entweder mit Chlorameisensäureisobutylester/Aceton, Natriumazid/Wasser oder mit Diphenylphosphorylazid / Tetrahydrofuran oder mit Xylol oder Methylenchlorid in Gegenwart einer der oben angegebenen Basen, vorzugsweise Triethylamin, bei -10°C bis Raumtemperatur umsetzt.

Die Verbindungen der allgemeinen Formeln (V) und (Va) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die Verbindungen der allgemeinen Formel (VII) sind größtenteils neu und können wie oben beschrieben hergestellt werden.

Die Verbindungen der allgemeinen Formel (la) sind neu und können beispielsweise wie unter [A], [B], [D] oder [E] beschrieben hergestellt werden.

Die Verbindungen der allgemeinen Formel (Ib), (Ic), (Id), (Ie) und (If) sind neu und können wie oben beschrieben hergestellt werden.

Die Verbindungen der allgemeinen Formel (VI) sind größtenteils bekannt oder neu und können beispielsweise hergestellt werden, indem man ausgehend von den freien Aminen (la) entweder mit dem Acetonid von Glycerinaldehyd in Methanol und in Anwesenheit von Natriumacetat / Natriumcyanoborhydrid oder von Natriumboranat und Methanol in einem Temperaturbereich von -20°C bis +40°C, bevorzugt von -10°C bis 20°C und Normaldruck umsetzt.

Die Einführung des Halogenatoms Y (Verbindungen der allgemeinen Formel (If)) erfolgt im Fall von Brom und Jod entweder mit elementarem Brom oder Jod oder in Anwesenheit eines Silber-Salzes, in einem der oben aufgeführten Lösemittel, vorzugsweise Methylenchlorid, Acetonitril oder Chloroform, in einem Temperaturbereich von -30°C bis +60°C, bevorzugt von 0°C bis +30°C und Normaldruck.

Als Silber-Salze eignen sich beispielsweise Silbertetrafluoroborat oder Silbertrifluormethansulfonat oder Silbertrifluoracetat.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration bei der mit bloßem Auge kein Wachstum zu erkennen war.

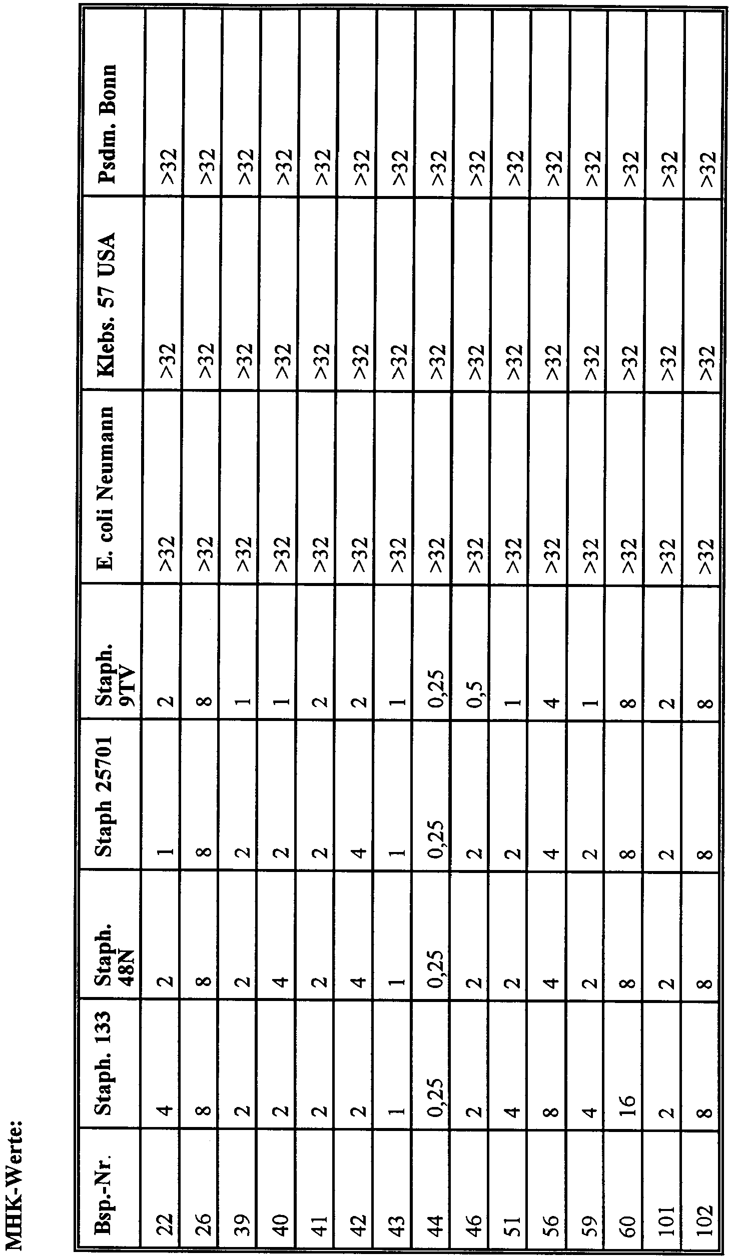

Die erfindungsgemäßen Verbindungen der allgemeinen Formeln (I), (la), (Ib), (Ic), (Id), (Ie) und (If) weisen bei geringer Toxizität ein breites antibakterielles Spektrum, speziell gegen gram-positive Bakterien sowie Mycobacterien, Corynebacterien, Haemophilus influenzae und anaerobae Keime auf. Diese Eigenschaften ermöglichen ihre Verwendung als chemotherapeutische Wirkstoffe in der Human-und Tiermedizin.

Die erfindungsgemäßen Verbindungen sind gegen ein breites Spektrum von Mikroorganismen wirksam. Mit ihrer Hilfe können gram-positive Bakterien und bakterienähnliche Mikroorganismen, wie Mycoplasmen, bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human-und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die neuen Verbindungen können in den üblichen Konzentrationen und Zubereitungen zusammen mit dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit anderen Antibiotika kombiniert werden.

### Anhang zum experimentellen Teil

### Liste der verwendeten Laufmittelgemische zur Chromatographie:

- I: Dichlormethan : Methanol
- II: Toluol : Ethylacetat
- III: Acetonitril : Wasser
- IV: Ethylacetat
- V: Petrolether : Ethylacetat

### Abkürzungen:

- Z: Benzyloxycarbonyl
- Boc: tert.Butyloxycarbonyl
- DMF: Dimethylformamid
- Ph: Phenyl
- Me: Methyl
- THF: Tetrahydrofuran
- CDI: Carbonyldiimidazol
- DCE: Dichlorethan

### Ausgangsverbindungen

### Beispiel I

### 4-Brom-benzo[b]thiophen-2-carbonsäureazid

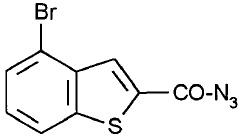

Zu einer auf 0°C gekühlten Lösung von 140 g (545 mmol) 4-Brombenzo[b]thiophen-2-carbonsäure [Indian J. Chem., Sect. B, 1984, S. 38-41] und 90ml (643 mmol) Triethylamin in 1120 ml Aceton tropft man langsam 91,2 ml (708 mmol) Chlorameisensäureisobutylester in 500 ml Aceton. Man rührt 45 min bei 0°C nach und tropft dann langsam 53,8 g (830 mmol) Natriumazid in 270 ml Wasser zu. Man rührt 1 Stunde bei 0°C nach und kippt den Ansatz auf 5 I Eiswasser. Man saugt den ausgefallenen Niederschlag ab, wäscht mit Wasser nach und trocknet an der Luft. Ausbeute: 116,7 g (76%)

### Beispiel II

### (5R)-3-[4-Brom-benzo[b]thiophenyl]-5-butyryloxy-methyl-oxazolidin-2-on

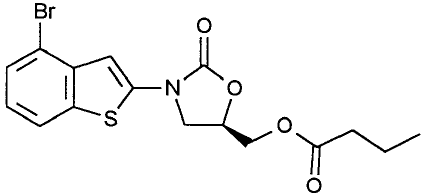

Eine Lösung von 3,2 g (38 mmol) Lithiumbromid und 8,3 g (38 mmol) Tributylphosphinoxid in 200 ml Xylol wird 1 h am Wasserabscheider gekocht. Nun wird in der Siedehitze eine Lösung von 105 g (372 mmol) der Verbindung aus Beispiel I und 52 ml (372 mmol) R(-)-Glycidylbutyrat in 300 ml Xylol möglichst schnell zugetropft (heftige Gasentwicklung). Nach beendeter Zugabe wird noch 10 min unter Rückfluß nachgerührt. Man läßt auf Raumtemperatur abkühlen und engt ein. Das Rohprodukt wird an Kieselgel mit Methylenchlorid chromatographiert.
Ausbeute: 44,4 g (30%)
¹H-NMR (D₆-DMSO, TMS): 7,89 (d, J = 7,5 Hz, 1 H); 7,58 (d, J = 7,5 Hz, 1 H); 7,19 (t, J = 7,5 Hz, 1 H); 6,88 (s, 1 H); 5,47 - 5,54 (m, 1 H); 4,72 (d, J = 13 Hz, 1 H); 4,28 - 4,48 (m, 2H); 4,0 - 4,1 (m, 1 H); 2,37 (t, J = 7,0 Hz, 2H); 1,55 (h, J = 7 Hz, 2H); 0,86 (t, J = 7 Hz, 3H).
MS (DCI): 398 (m⁺, 95%), 400 (M⁺+2, 100%)

### Beispiel III

### 2-Benzyloxycarbonylamino-5-brom-thiazol

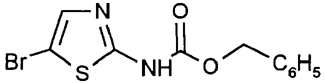

Zu einer Lösung von 25 g (96 mmol) 2-Amino-5-bromthiazol in 100 ml Dioxan und 190 ml ges. NaHCO₃-Lösung werden unter Eiskühlung 15,1 ml (106 mmol) Chlorameisensäurebenzylester langsam zugetropft. Man rührt über Nacht bei RT nach, dampft das Dioxan im Vakuum ab, saugt den ausgefallenen Feststoff ab und wäscht mit Wasser und mit Petrolether nach. Der Rückstand wird an Kieselgel mit Dichlormethan / Methanol (50:1) als Eluens gereinigt.
Ausbeute: 17 g (58%)
¹H-NMR (D₆-DMSO, TMS): 12,62 (s, 1 H); 7,47 (s, 1 H); 7,40 (m, 5H); 5,23 (s, 2H).

### Beispiel IV

### 2-Benzyloxycarbonylamino-4-phenylthiazol

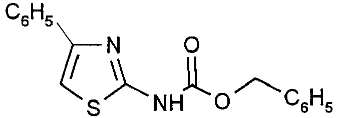

Das Produkt wird ausgehend von 2-Amino-4-phenylthiazol [J. Med. Chem. 26, 1158 (1983)] analog Beispiel III erhalten. ¹H-NMR (D₆-DMSO, TMS): 12,00 (s, 1 H); 7,88 (d, J = 8 Hz, 2H); 7,60 (s, 1 H); 7,37 (m, 8H); 5,26 (s, 2H).

### Beispiel V

### 6-Brom-2-n-Butyloxycarbonylamino-benzo[b]thiophen

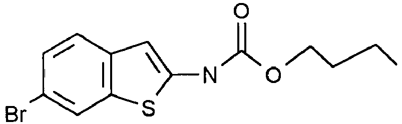

In 1,6 I siedendes n-Butanol werden portionsweise 134,4 g (451 mmol) 6-Brom-benzo[b]thiophen-2-carbonsäureazid (dargestellt analog Beispiel I) eingetragen (Vorsicht, heftige Gasentwicklung). Nach beendeter Zugabe wird noch 10 min. unter Rückfluß gekocht, dann auf RT abgekühlt, und das n-Butanol am Rotationsverdampfer abgezogen. Der Rückstand wird in 1 I Petrolether / Ether (8/2) 1 Stunde lang verrührt und abgesaugt.
Ausbeute: 112 g (76%)
Smp.: 115°C
¹H-NMR (D₆-DMSO, TMS): 11,04 (s, 1 H); 8,08 (d, J = 2 Hz, 1 H); 7,58 (d, J = 7 Hz, 1 H); 7,4 (dd, J = 7 Hz, J = 2 Hz, 1 H); 6,8 (s, 1 H); 4,16 (t, J = 6,5 Hz, 2H); 1,63 (q, J = 6,5 Hz, 2H); 1,38 (h, J = 6,5 Hz, 2H); 0,92 (t, J = 6,5 Hz, 3H).

### Herstellungsbeispiele

### Beispiel 1

### (5R)-3-[4-Brom-benzo[b]-thiophenyl]-5-hydroxy-methyloxazolidin-2-on

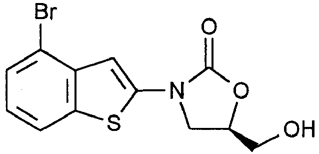

44,4 g (109,5 mmol) der Verbindung aus Beispiel II werden mit 7,14 g (21,9 mmol) Caesiumcarbonat versetzt, in 500 ml Methanol gelöst und über Nacht bei Raumtemperatur gerührt. Man engt ein, verrührt den Rückstand mit 500 ml Petrolether und saugt ab. Der Niederschlag wird gut mit Wasser und Petrolether nachgewaschen und getrocknet.
Ausbeute: 22 g (61 %)
Smp.: 203°C
¹H-NMR (D₆-DMSO, TMS): 7,9 (d, J = 7,5 Hz, 1 H); 7,55 (d, J = 7,5 Hz, 1 H); 7,17 (t, J = 7,15 Hz, 1 H); 6,7 (s, 1 H); 5,33 (br, 1 H); 4,83 - 4,93 (m, 1 H); 4,23 (t, J = 9,5 Hz, 1 H); 4,0 (dd, J = 9,5 Hz, 6,5 Hz, 1 H); 3,58 - 3,80 (m, 2H). α_{D}²⁰ = -76,7°(c= 0,9, DMSO)

### Beispiel 2

### (5R)-3-(5-Brom-2-thiazolyl)-5-hydroxymethyl-oxazolidin-2-on

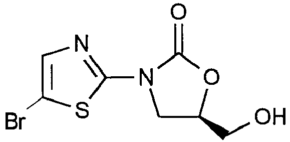

15,6 g (49,5 mmol) der Verbindung aus Beispiel III werden in 125 ml abs. Tetrahydrofuran suspendiert. Man fügt bei - 78°C 59,6 ml einer 1 M Lösung von Lithium-bis-trimethylsilylamid in Tetrahydrofuran erwärmt für 15 min auf 0°C und kühlt wiederum auf -78°C. Dann werden 13,9 ml (99,5 mmol) (R)-Glycidylbutyrat zugegeben und 18 h nachgerührt, wobei die Reaktionslösung langsam auf RT kommt. Der entstandene Niederschlag wird abgesaugt, das Filtrat mit Dichlormethan verdünnt, mit ges. Ammoniumchloridlösung gewaschen und die wäßrige Phase dreimal mit Dichlormethan gewaschen. Die vereinigten organischen Phasen werden getrocknet (MgSO₄) und eingeengt. Der erhaltene Rückstand sowie der oben erhaltene produkthaltige Niederschlag werden an Kieselgel mit Toluol / Essigester (5:1 ≧ 1:1) chromatographiert. Man erhält 1,91 g Produkt sowie 6,06 g des Butyrats des Produktes. Dieser Ester wird in 24 ml Methanol gelöst, mit 1,16 ml (24 mol) Hydrazinhydrat versetzt und 4 h bei RT gerührt. Man engt ein und erhält eine weitere Fraktion des Produktes. Alle produkthaltigen Fraktionen werden mit Ether verrieben und der erhaltene Feststoff wird abgesaugt und getrocknet.
Ausbeute: 3,07 g (23,5%)
¹H-NMR (D₆-DMSO, TMS): 7,59 (s, 1 H); 5,27 (t, J = 5 Hz, 1 H); 4,87 (m, 1 H); 4,18 (t, J = 9 Hz, 1 H); 3,94 (dd, J = 9,6 Hz, 1 H); 3,72 (m, 1 H); 3,57 (m, 1 H).

### Beispiel 3

### (5R)-3-(4-Phenyl-2-thiazolyl)-5-hydroxy-methyloxazolidin-2-on

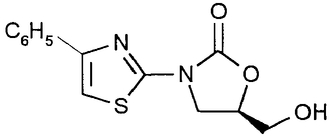

10 g (32,2 mmol) der Verbindung aus Beispiel IV in 80 ml absolutem Tetrahydrofuran werden bei -78°C mit 11,6 ml 2,5 N n-Butyllithium-Lösung in Hexan versetzt. Man läßt auf -30°C aufwärmen, kühlt dann wieder auf -78°C und fügt 4,05 ml (29 mmol) (R)-Glycidylbutyrat zu. Man rührt 18 h nach, wobei die Reaktionslösung auf RT aufwärmt. Zur Aufarbeitung wird mit Dichlormethan verdünnt, mit gesättigter Ammoniumchlorid-Lösung gewaschen, und die wäßrige Phase dreimal mit Dichlormethan extrahiert. Die organischen Phasen werden vereinigt, getrocknet (MgSO₄) und eingeengt. Der Rückstand wird an Kieselgel gereinigt mit Toluol/Essigester (20:1 > 1:1) als Eluens.
Ausbeute: 4,75 g (53,5%)
Fp.: 156°C (Ether)
¹H-NMR (D₆-DMSO, TMS): 7,93 (d, J = 8 Hz, 2H); 7,71 (s, 1 H); 7,5 - 7,30 (m, 3H); 5,28 (t, J = 5 Hz, 1 H); 4,77 (m, 1 H); 4,32 (t, J = 9 Hz, 1 H); 4,13 (dd, J = 9,6 Hz, 1 H); 3,73 (m, 1 H); 3,62 (m, 1 H).
[α]_{D}²⁰ = -90,2° (c=0,5, DMSO)
MS (EI): m/e = 276 (M⁺), 134 (100%)

### Beispiel 4

### (5R)-3-[6-Brom-benzo[b]-thiophenyl]-5-hydroxy-methyloxazolidin-2-on

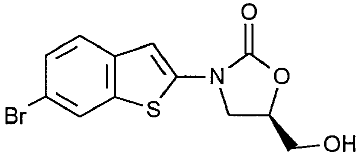

112 g (341 mmol) der Verbindung aus Beispiel V werden in 1 I THF gelöst, mit 10 mg 1,10-Phenanthrolin-Hydrat versetzt und auf -70°C gekühlt. Nun werden langsam ca. 136 ml 2,5 N-n-Butyllithium-Lösung in Hexan bis zum Farbumschlag nach rot zugetropft. Anschließend werden 48,9 ml (350 mmol) (R)-Glycidylbutyrat zugetropft. Man läßt auf Raumtemperatur kommen, versetzt mit gesättigter Ammoniumchlorid-Lösung, trennt die organische Phase ab und extrahiert die wäßrige Phase zweimal mit Essigester. Die vereinigten organischen Phasen werden getrocknet (Na₂SO₄) und eingeengt. Der Rückstand wird in Ether verrührt, abgesaugt und getrocknet.
Ausbeute: 97,4 g (87%)
Smp.: 246°C
α_{D}²⁰ = -54,2° (c = 0,9, DMSO)
¹H-NMR (D₆-DMSO, TMS): 8,15 (d, J = 2 Hz, 1 H); 7,63 (d, J = 7 Hz, 1 H); 7,47 (dd, J = 7 Hz, J = 2 Hz, 1 H); 6,8 (s, 1 H); 5,28 (br, 1 H); 4,7 - 4,95 (m, 1 H); 4,19 (t, J = 9,5 Hz, 1 H); 3,92 (dd, J = 9,5 Hz, 6,5 Hz, 1 H); 3,55 - 3,80 (m, 2H).

In Analogie zur Vorschrift des Beispiels 4 werden die in Tabelle 1 aufgeführten Verbindungen hergestellt:

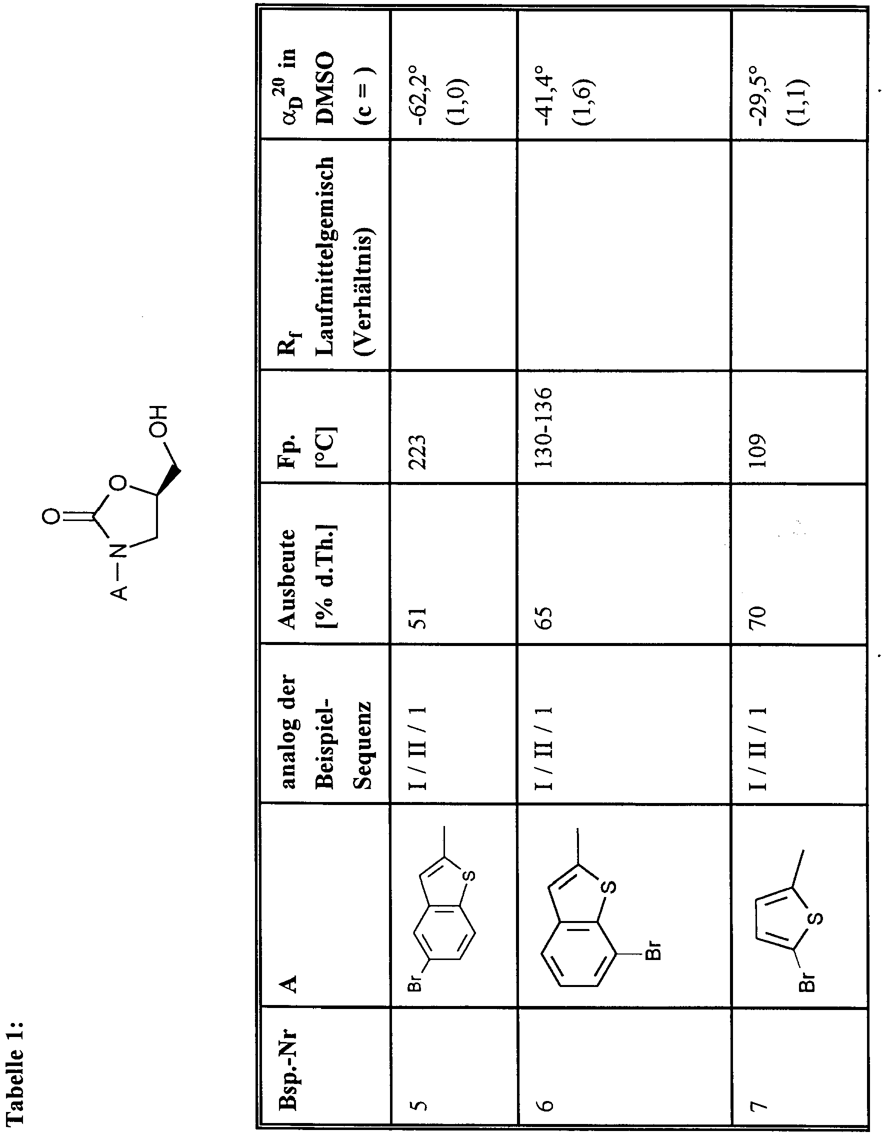

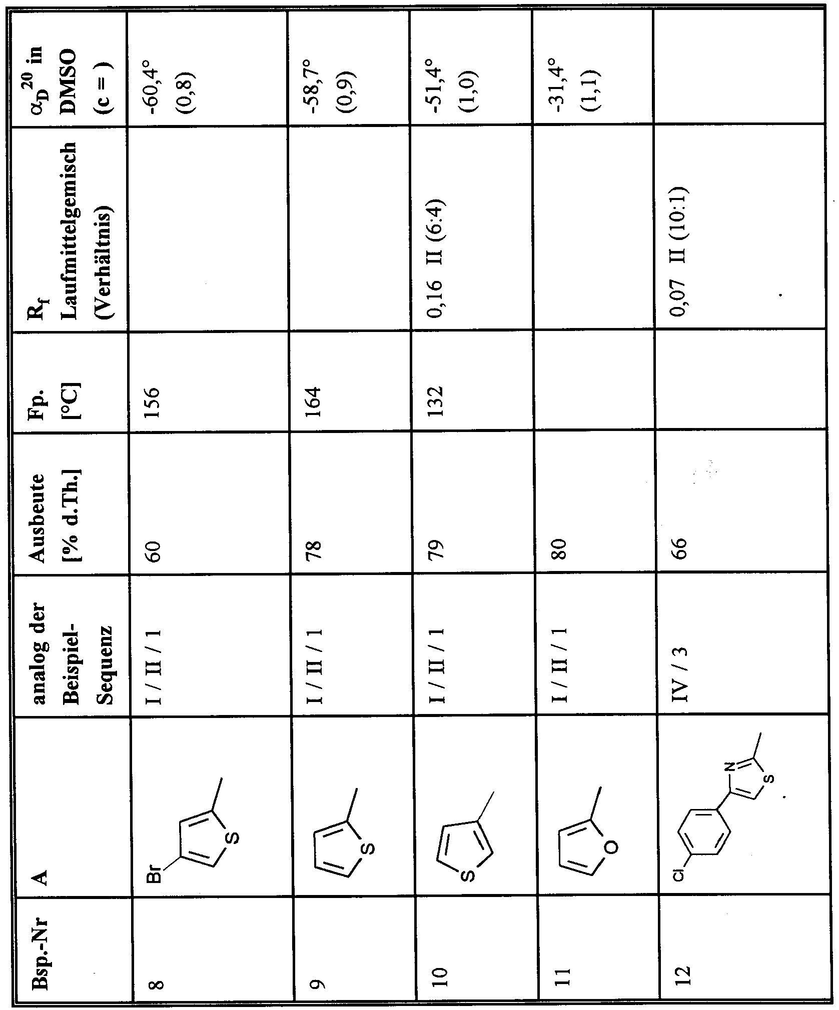

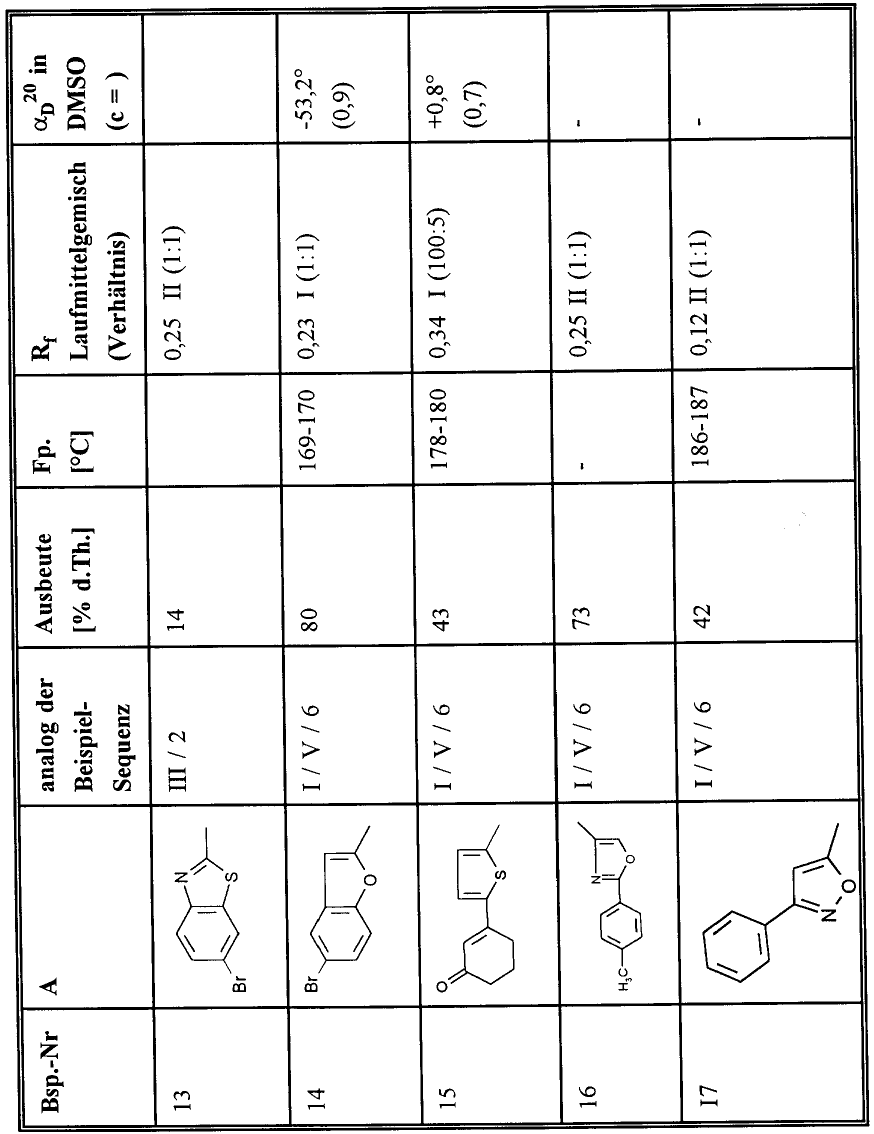

### Beispiel 18

### (5R)-3-[4-Brom-benzo[b]thiophenyl]-5-methan-sulfonyloxymethyl-oxazolidin-2-on

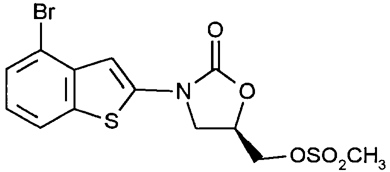

Eine Lösung von 22 g (67,3 mmol) der Verbindung aus Beispiel 1 und 15,5 ml (113 mmol) Triethylamin in 250 ml Methylenchlorid wird auf -10°C abgekühlt und langsam mit 8 ml (107 mmol) Methansulfonsäurechlorid versetzt. Es wird 1 h bei -10°C nachgerührt und auf Eiswasser gekippt. Nach Abtrennen der organischen Phase wird diese nacheinander mit verdünnter HCl, ges. NaHCO₃ und H₂O je 1 mal extrahiert. Man trocknet die organische Phase mit Natriumsulfat und engt ein.
Ausbeute: 25 g (92%)
¹H-NMR (D₆-DMSO, TMS): 7,92 (d, J = 7,5 Hz, 1 H); 7,58 (d, J = 7,5 Hz, 1 H); 7,18 (t, J = 7,5 Hz, 1 H); 6,72 (s, 1 H); 5,11 -5,28 (m, 1 H); 4,57 (d, J = 5 Hz, 2H); 4,36 (t, J = 15 Hz, 1 H); 4,02 (dd, J = 15 Hz, J = 6 Hz, 1 H); 3,28 (s, 3H).

### Beispiel 19

### (5R)-3-[4-Brom-benzo[b]thiophenyl]-5-azidomethyl-oxazolidin-2-on

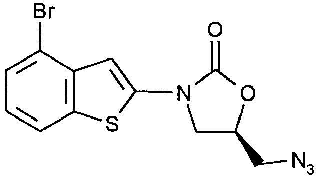

25 g (62 mmol) der Verbindung aus Beispiel 18 werden in 250 ml DMF gelöst und mit 4,4 g (67 mmol) Natriumazid versetzt. Die so erhaltene Reaktionsmischung wird 14 h bei 70°C gerührt. Man läßt auf Raumtemperatur abkühlen und kippt auf 2 I Eiswasser. Man saugt vom ausgefallenen Feststoff ab, wäscht mit Wasser und Petrolether nach und trocknet an der Luft.
Ausbeute: 20,3 g (93%)
Smp.: 115°C
α_{D}²⁰ = -180,8° (DMSO, c = 0,5)
¹H-NMR (D₆-DMSO, TMS): 7,93 (d, J = 7,5 Hz, 1 H); 7,58 (d, J = 7,5 HZ, 1 H); 7,19 (t, J = 7,5 Hz, 1 H); 6,73 (s, 1 H); 5,0 -5,14 (m, 1 H); 4,3 (t, J = 10 Hz, 1 H); 3,97 (dd, J = 10 Hz, J = 6 Hz, 1 H); 3,8 (d, J = 5 Hz, 2H).
MS (DC): 353 (M⁺, 98%); 355 (M⁺2, 100%)

### Beispiel 20

### (5R)-3-[4-Brom-[b]thiophenyl]-5-aminomethyloxazolidin-2-on Hydrochlorid

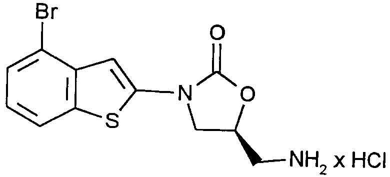

20 g (57 mmol) der Verbindung aus Beispiel 19 werden in 60 ml Ethylenglykoldimethylether gelöst und auf 50°C erwärmt. Man tropft langsam 8 ml (68 mmol) Trimethylphosphit zu (Gasentwicklung), erwärmt nach beendeter Zugabe auf 90°C und rührt 2 h bei 90°C nach. Nun tropft man 10,7 ml (65 mmol) 6 N HCl zu und rührt wieder 2 h bei 90°C nach. Man läßt auf RT abkühlen, engt ein und verrührt den Rückstand mit heißem Ethanol. Man läßt auf RT abkühlen und saugt vom Niederschlag ab. Der Niederschlag wird mit wenig Ethanol und viel Petrolether nachgewaschen und am Hochvakuum getrocknet.
Ausbeute: 15 g (73%)
Smp.: > 240°C
¹H-NMR (D₆-DMSO, TMS): 7,92 (d, J = 7,5 Hz, 1 H); 7,56 (d, J = 7,5 Hz, 1 H); 7,19 (t, J = 7,5 Hz, 1 H); 6,68 (s, 1 H); 5,05
- 5,22 (m, 1 H); 4,38 (t, J = 10 Hz, 1 H); 4,04 (dd, J = 10 Hz, J = 6 HZ, 1 H); 3,3 (d, J = 6 Hz, 2H)
α_{D}²⁰ = -60,7° (c = 0,9, DMSO)

### Beispiel 21

### (5S)-3-[4-Brom-benzo[b]thiophenyl]-5-acetyl-aminomethyl-oxazolidin-2-on

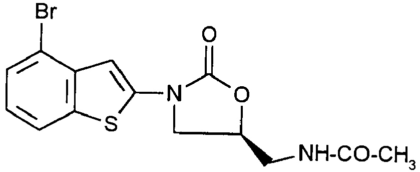

15 g (41 mmol) der Verbindung aus Beispiel 20 werden mit 150 ml Methylenchlorid und 13,9 ml (103 mmol) Triethylamin versetzt. Die so erhaltene Reaktionslösung wird unter Rühren auf 0°C gekühlt und langsam mit 3,9 ml (57 mmol) Triethylamin versetzt. Die so erhaltene Reaktionslösung wird unter Rühren auf 0°C gekühlt und langsam mit 3,9 ml (57 mmol) Acetylchlorid versetzt. Man rührt 2 h bei 0°C nach, verdünnt mit 200 ml Wasser und 150 ml Methylenchlorid. Man trennt die organische Phase ab, wäscht die wäßrige Phase 1 mal mit Methylenchlorid nach und trocknet die vereinigten organischen Phasen mit Natriumsulfat. Man engt ein, verrührt den Rückstand mit 200 ml Ether und saugt ab.
Ausbeute: 12,2 g (83% d.Th.)
Smp.: 177°C
¹H-NMR (D₆-DMSO, TMS): 8,28 (t, J = 6,3 Hz, 1 H); 7,92 (d, J = 7,5 Hz; 1 H); 7,58 (d, J = 7,5 Hz, 1 H); 7,19 (t, J = 7,5 Hz, 1 H); 6,68 (s, 1 H); 4,83 - 4,96 (m, 1 H); 4,29 (t, J = 9,5 Hz, 1 H); 3,9 (dd, J = 9,5 Hz, J = 6 Hz, 1 H); 3,43 - 3,52 (m, 2H); 1,87 (s, 3H).
MS (DCI): 370 (M⁺-1, 40%)

In Analogie zu den Vorschriften der Beispiele 18-21 werden die in der Tabelle 2 aufgeführten Verbindungen hergestellt:

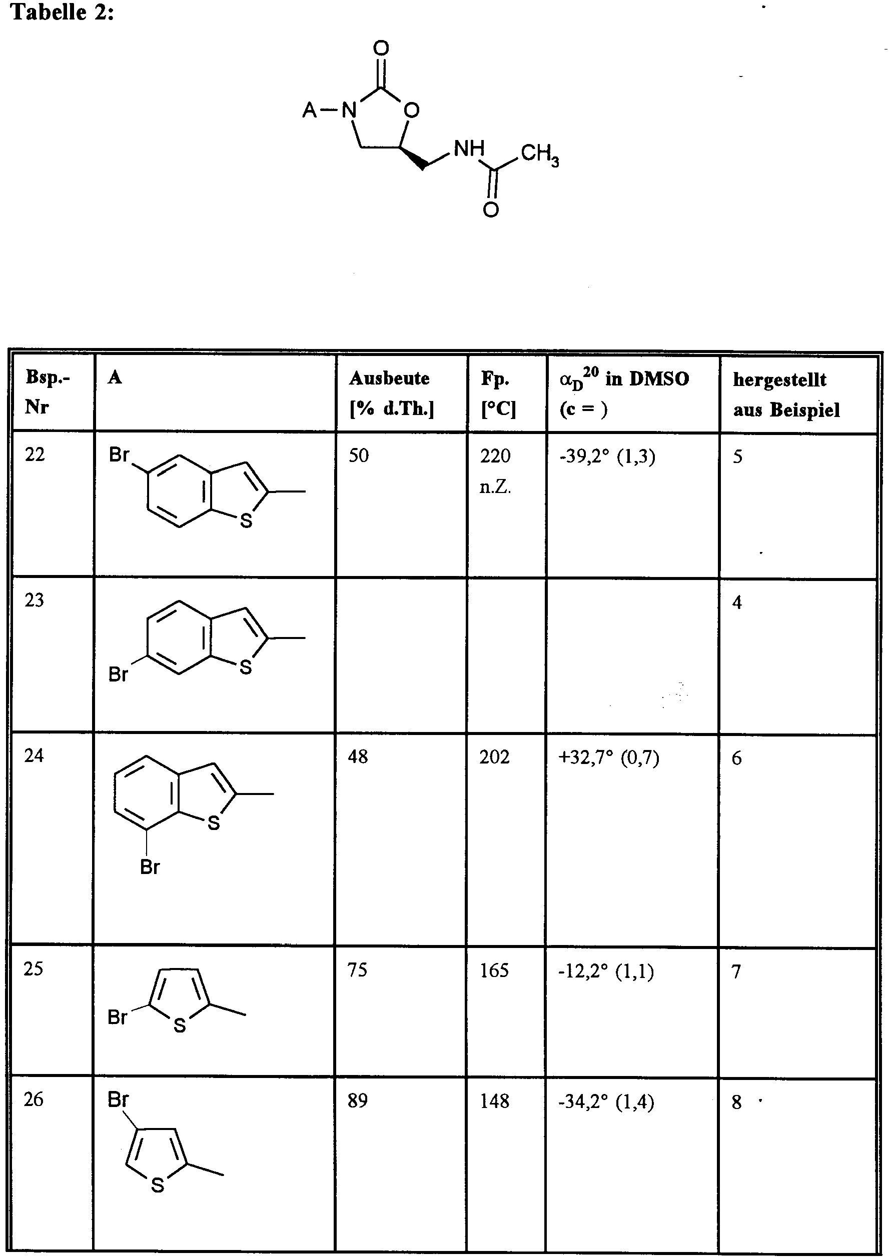

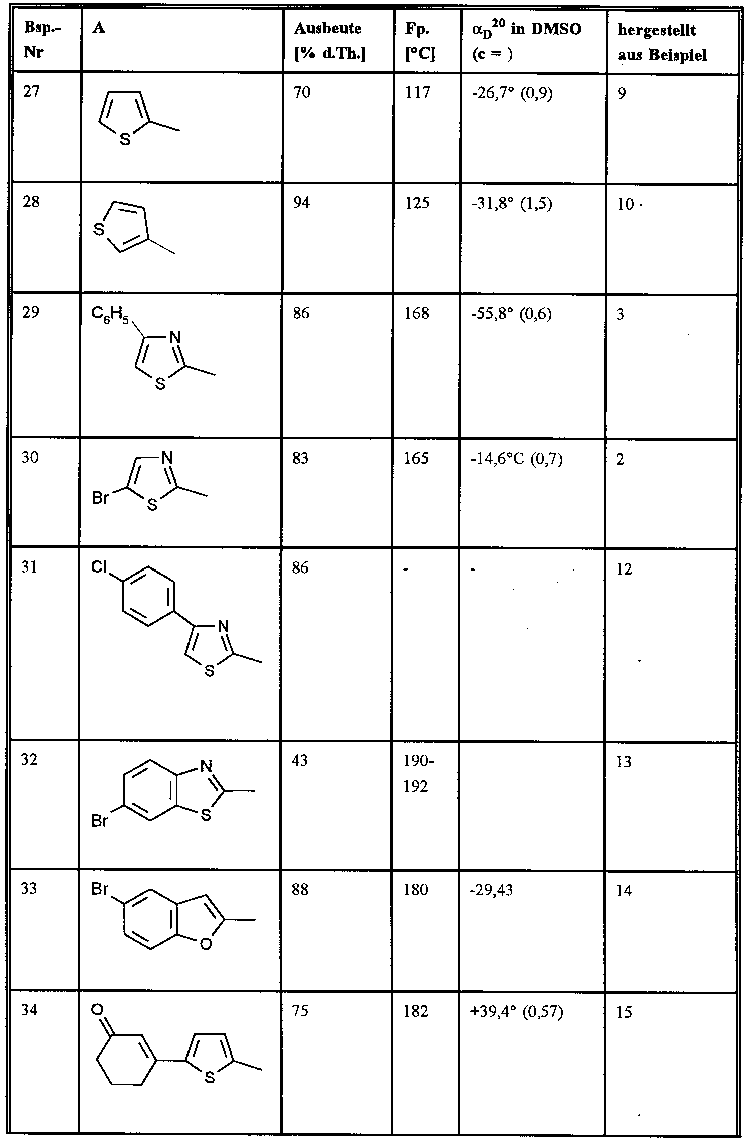

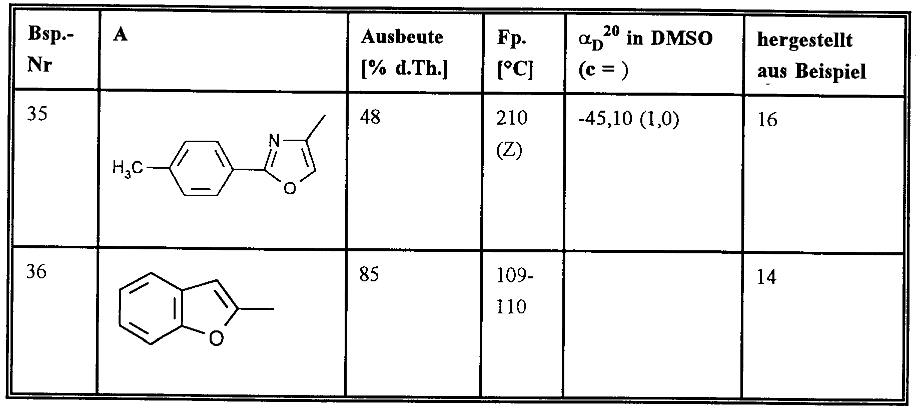

### Beispiel 37

### (5S)-3-[2-(5-Bromthiophenyl)]-5-acetylaminomethyl-oxazolidin-2-on

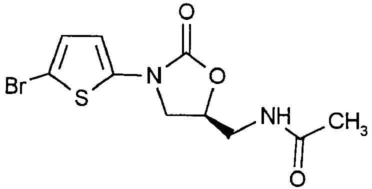

5,6 g (23,4 mmol) der Verbindung aus Beispiel 28 werden in 65 ml Chloroform und 45 ml Acetonitril gelöst und auf 0°C gekühlt. Man tropft nun eine Lösung von 0,6 ml Brom in 5 ml Chloroform langsam zu und laßt über Nacht unter Rühren auf RT kommen. Man engt ein, nimmt in 200 ml Methylenchlorid auf und wäscht je einmal mit verdünnter Thiosulfatlösung und Wasser. Man trocknet die organische Phase mit Natriumsulfat, engt ein und chromatographiert an Kieselgel mit Petrolether zu Essigester 1:1.
Ausbeute: 4,82 g (65%)
¹H-NMR (D₆-DMSO, TMS): 8,24 (t, J = 6,5 Hz, 1 H); 7,05 (d, J = 5 Hz, 1 H); 6,34 (d, J = 5 Hz, 1 H); 4,78 - 4,92 (m, 1 H); 4,1 (t, J = 8 Hz, 1 H); 3,7 (dd, J = 9 Hz, J = 7 Hz, 1 H); 3,4 - 3,48 (m, 2H); 1,8 (s, 3H).
MS (DCI): 316 (M⁺+1, 20%)

### Beispiel 38

### (5S)-3-[2-(5-Iodthiophenyl)]-5-acetylaminomethyl-oxazolidin-2-on

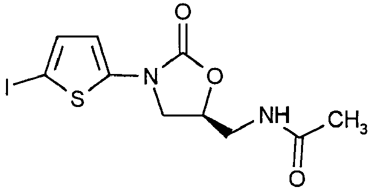

5 g (21 mmol) der Verbindung aus Beispiel 28 werden in 60 ml Chloroform und 40 ml Acetonitril gelöst und mit 6,2 g (28 mmol) Silbertrifluoracetat versetzt. Man gibt nun 5,6 g (22 mmol) Jod portionsweise zu und rührt 48 h bei Raumtemperatur nach. Man engt ein, nimmt in Methylenchlorid auf und wäscht je einmal mit verdünnter Natriumthiosulfatlösung und Wasser. Man trocknet die organische Phase mit Natriumsulfat, engt ein und chromatographiert an Kieselgel mit Essigester zu Petrolether 3:7.
Ausbeute: 5,7 g (75%)
Smp.: >120°C, n.Z.
¹H-NMR (D₆-DMSO, TMS): 8,25 (br, 1 H); 7,14 (d, J = 5 Hz, 1 H); 6,26 (d, J = 5 Hz, 1 H); 4,7 - 4,9 (m, 1 H); 4,08 (t, J = 9 Hz, 1 H); 3,68 (dd, J = 9 Hz, J = 6 Hz, 1 H); 3,38 - 3,5 (m, 2H); 1,8 (s, 3H).
MS (DCI): 367 (M⁺+1, 100%)

### Beispiel 39

### (5S)-3-{2[5-(4-Methylphenyl)]thiophenyl}-5-acetyl-aminomethyl-oxazolidin-2-on

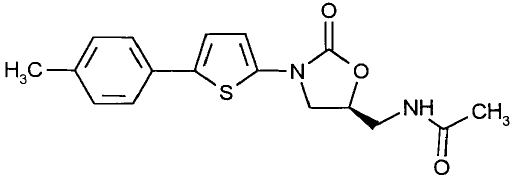

510 mg (1,6 mmol) der Verbindung aus Beispiel 25 und 286 mg (2,1 mmol) 4-Methylphenylboronsäure werden in 10 ml Toluol gelöst und mit 55 mg (0,048 mmol) Pd(P(C₆H₅)₃)₄ versetzt. Man kocht die so erhaltene Lösung 1 Stunde unter Rückfluß und gibt dann 2,2 ml 2 M Na₂CO₃ zu. Nun wird 16 h unter Rückfluß gekocht, auf RT abgekühlt, eingeengt und an Kieselgel chromatographiert (Petrolether/Essigester 2:8).
Ausbeute: 240 mg (45%)
Smp.: 215°C u.Z.
α_{D}²⁰: +6,4° (DMSO, c = 0,96)
MS (DCI): 331 (M⁺+1, 100%)
¹H-NMR (DCI): 8,27 (t, J = 7 Hz, 1 H); 7,48 (d, J = 8 Hz, 2H); 7,22 (d, J = 5 Hz, 1 H); 7,18 (d, J = 8 Hz, 2H); 6,5 (d, J = 5 Hz, 1 H); 4,77 - 4,90 (m, 1 H); 4,14 (t, J = 9 Hz, 1 H); 3,74 (dd, J = 9 Hz, J = 6 Hz, 1 H); 3,4 - 3,5 (m, 2H); 2,3 (s, 3H); 1,83 (s, 3H).

### Beispiel 40

### (5S)-3-{2-[5-(2-Formylphenyl)]thiophenyl}-5-acetylamino-methyl-oxazolidin-2-on

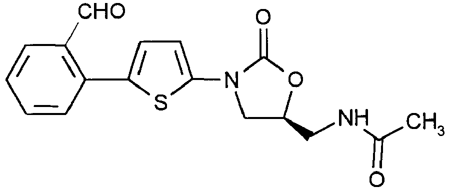

510 mg (1,6 mmol) der Verbindung aus Beispiel 25 und 315 mg (2,1 mmol) 2-Formyl-phenylboronsäure (J. Org. Chem. 57 (3), 1992, S. 1015-18) werden in 10 ml THF gelöst, mit 55 mg (0,048 mmol) Pd(P(C₆H₅)₃)₄ versetzt und 1 h unter Rückfluß gekocht. Man gibt 2,2 ml 2 M Na₂CO₃ Lösung zu und kocht 16 h unter Rückfluß, kühlt auf RT ab, engt ein und chromatographiert an Kieselgel (Petrolether / Essigester 4:6).
Ausbeute: 434 mg (79%)
Smp.: 151°C
α_{D}²⁰ = -6,3° (DMSO, c = 0,98)
MS (DCI): 345 (M⁺+1, 30%)
¹H-NMR (D₆-DMSO, TMS): 10,1 (s, 1 H); 8,29 (t, J = 7 Hz, 1 H); 7,88 -7,93 (m, 1 H); 7,7 - 7,78 (m, 1 H); 7,5 - 7,63 (m, 1 H); 7,08 (d, J = 4 Hz, 1 H); 6,67 (d, J = 4 Hz, 1 H); 4,7 - 4,95 (m, 1 H); 4,19 (t, J = 9 Hz, 1 H); 3,8 (dd, J = 9 Hz, J = 6 Hz, 1 H); 3,48 (t, J = 6 Hz, 2H); 1,87 (s, 3H).

### Beispiel 41

### (5S)-3-[5-(2-Formyl-3-thienyl)-2-thiazolyl]-5-acetylaminomethyloxazolidin-5-on

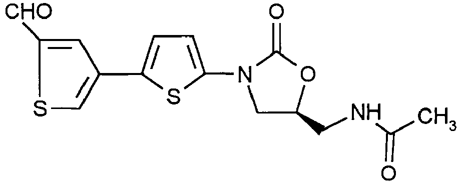

510 mg (1,6 mmol) der Verbindung aus Beispiel 25 und 320 mg (2,1 mmol) 4-(2-Formyl)thiophenboronsäure werden in 10 ml THF gelöst, mit 55 mg (0,048 mmol) Pd(PPh₃)₄ versetzt und 1 h unter Rückfluß gekocht. Man gibt 2,2 ml 2 M Na₂CO₃-Lösung zu und kocht 16 h unter Rückfluß, kühlt auf RT ab, filtriert ab, wäscht den Rückstand mit Wasser, THF und Ether und trocknet.
Ausbeute: 270 mg (48%)
α_{D}²⁰ = 4,3° (DMSO, C=1,0)
Smp.: > 205°C u.Z.
MS (DCI): 351 (M⁺+1, 19%)
¹H-NMR (D₆-DMSO, TMS): 9,95 (s, 1 H); 8,34 (s, 1 H); 8,28 (t, J = 7 Hz, 1 H); 8,17 (s, 1 H); 7,30 (d, J = 4 Hz, 1 H); 6,53 (d, J = 4 Hz, 1 H); 4,7 - 4,95 (m, 1 H); 4,15 (t, J = 9 Hz, 1 H); 3,77 (dd, J = 9 Hz, J = 6 Hz, 1 H); 3,48 (t, J = 6 Hz, 2H); 1,85 (s, 3H).

In Analogie zu der Vorschrift der Beispiele 39 - 41 werden die in der Tabelle 3 aufgeführten Verbindungen hergestellt:

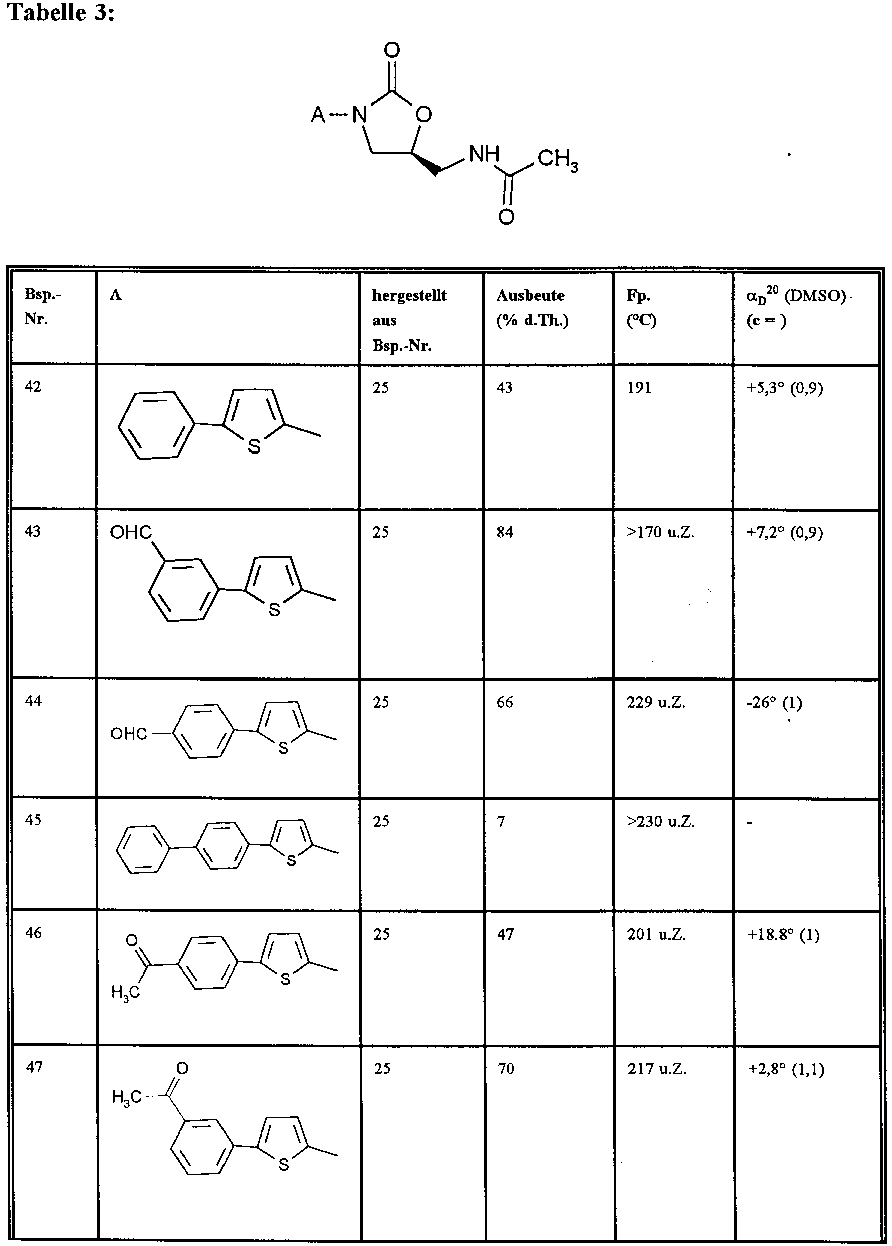

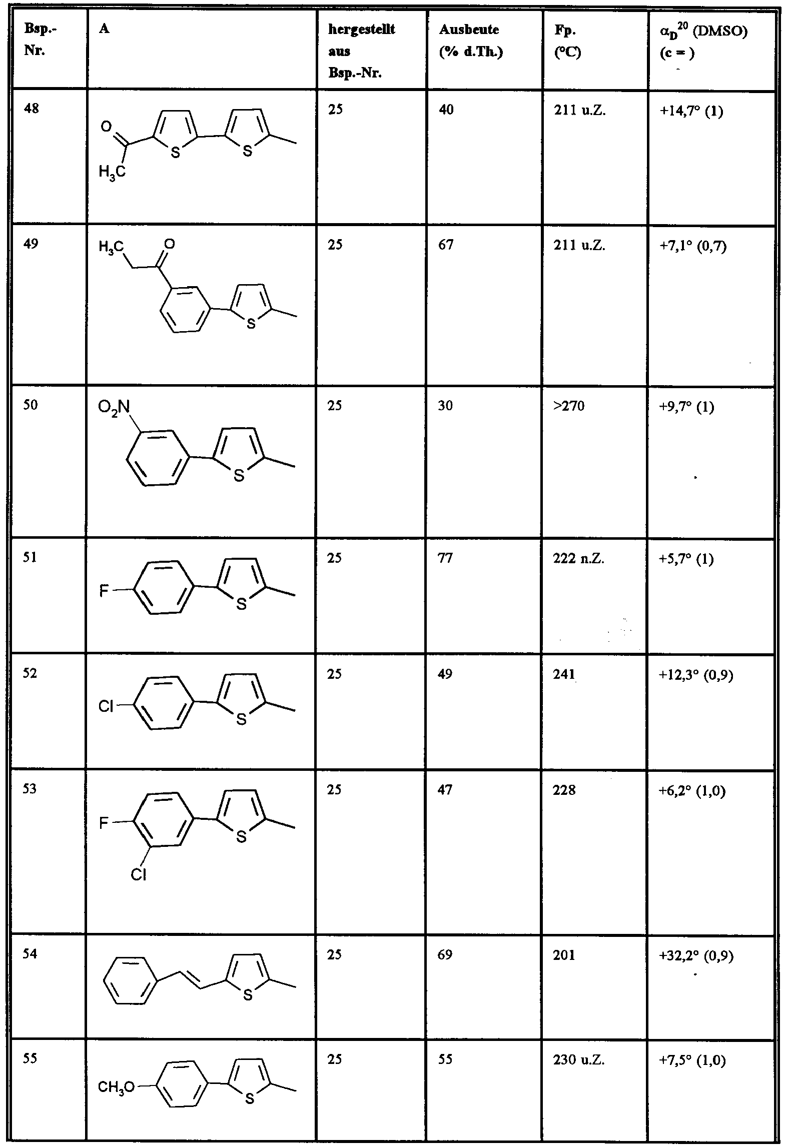

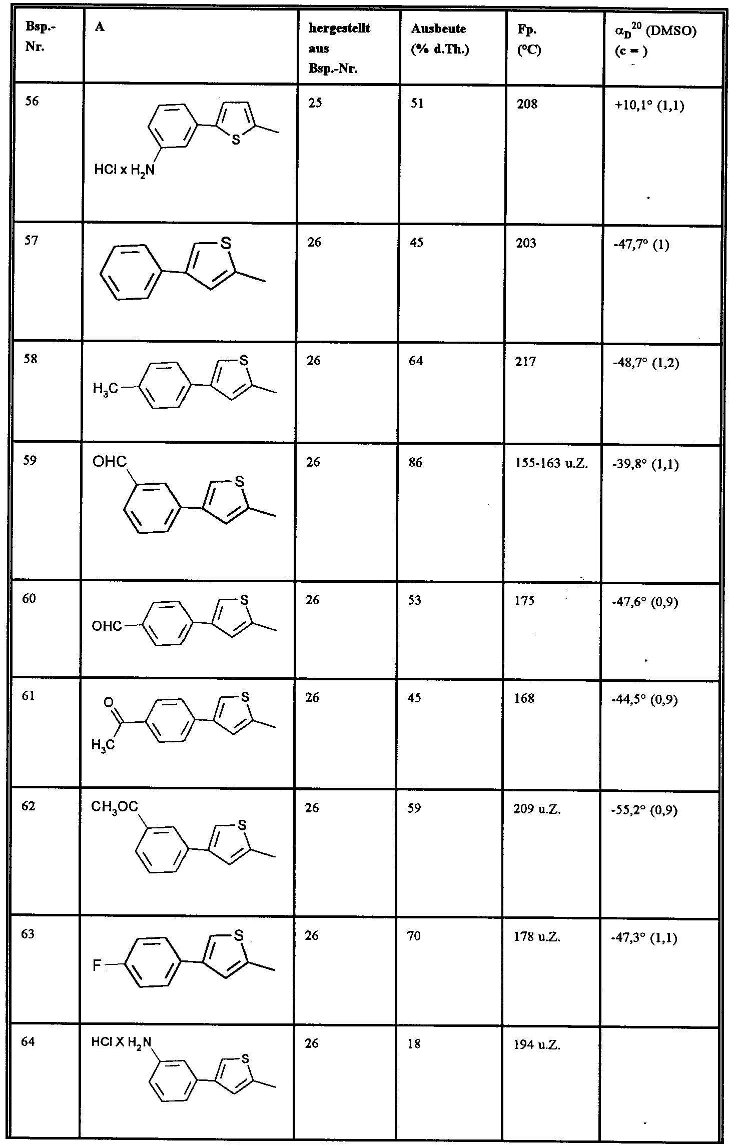

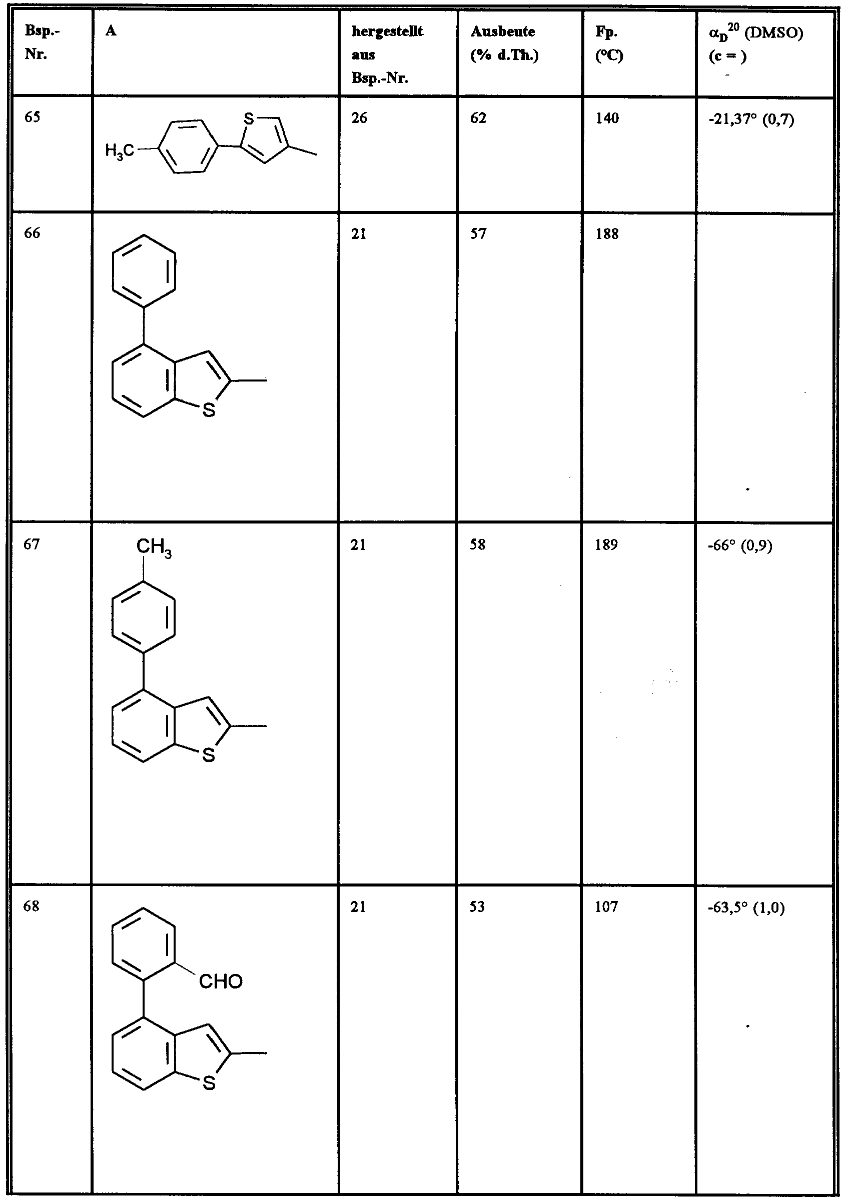

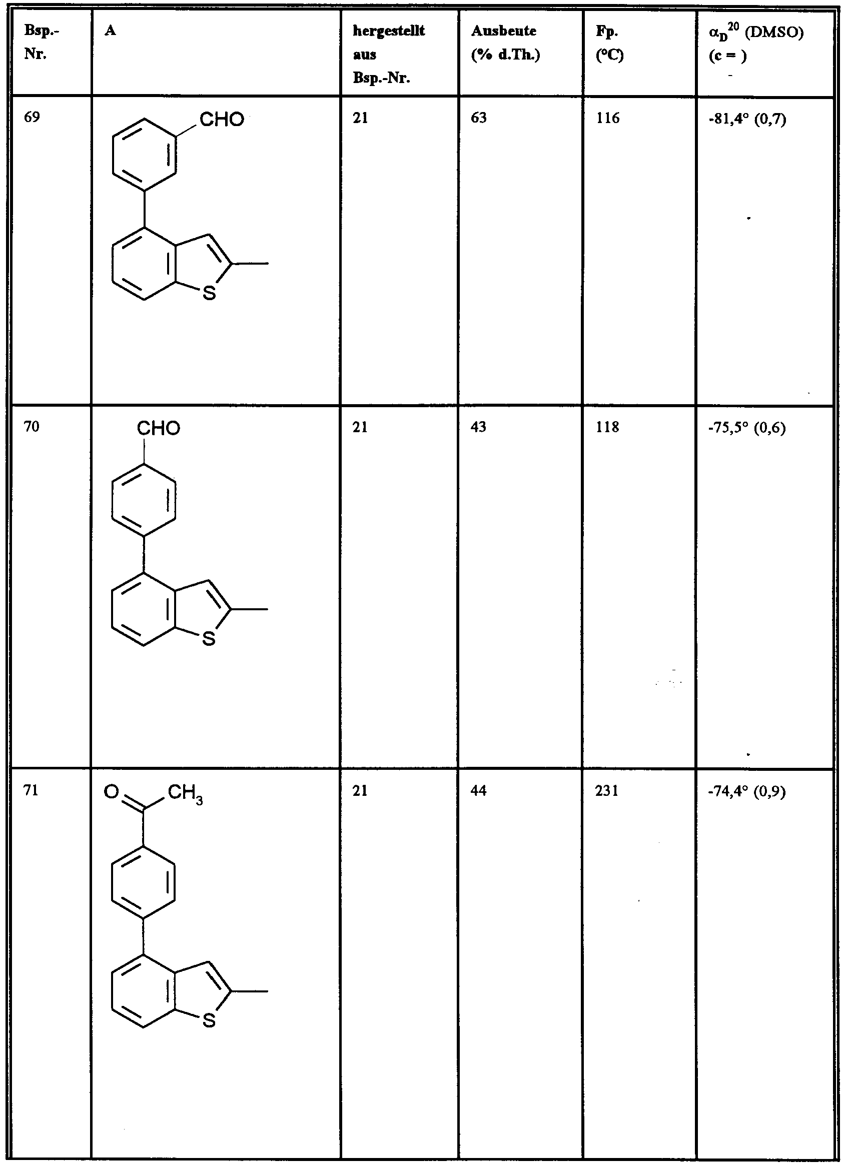

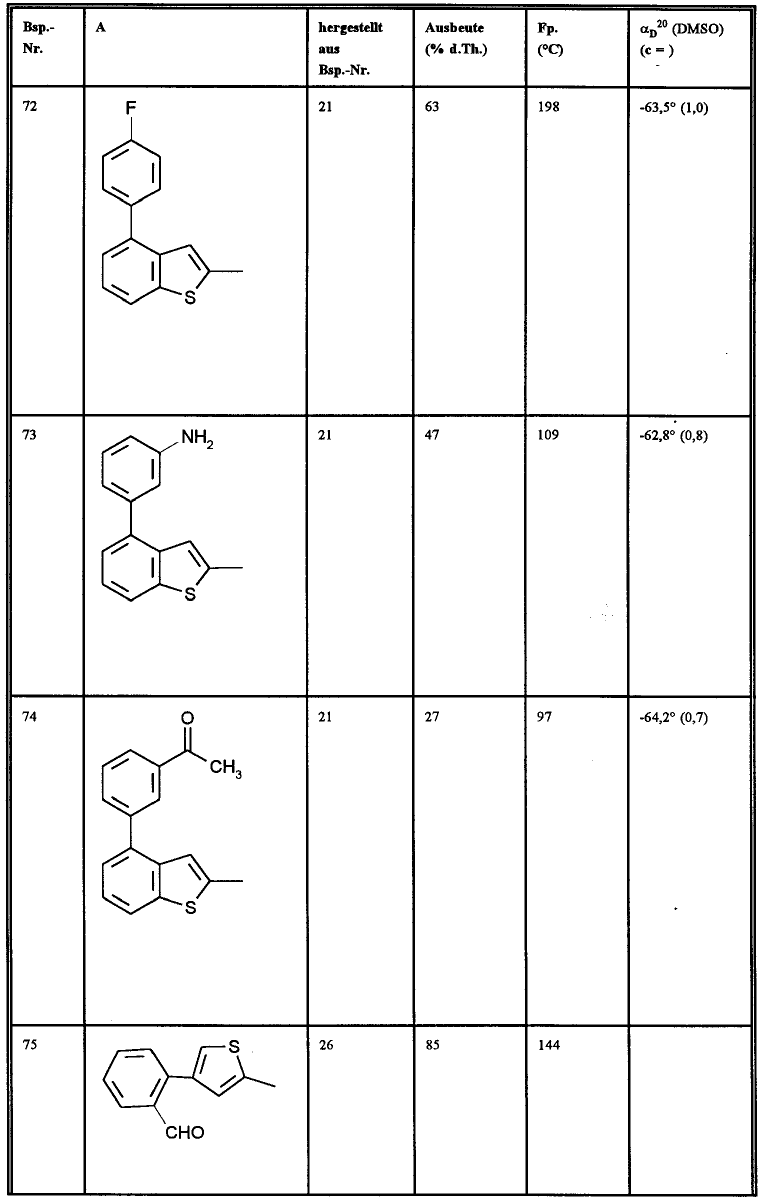

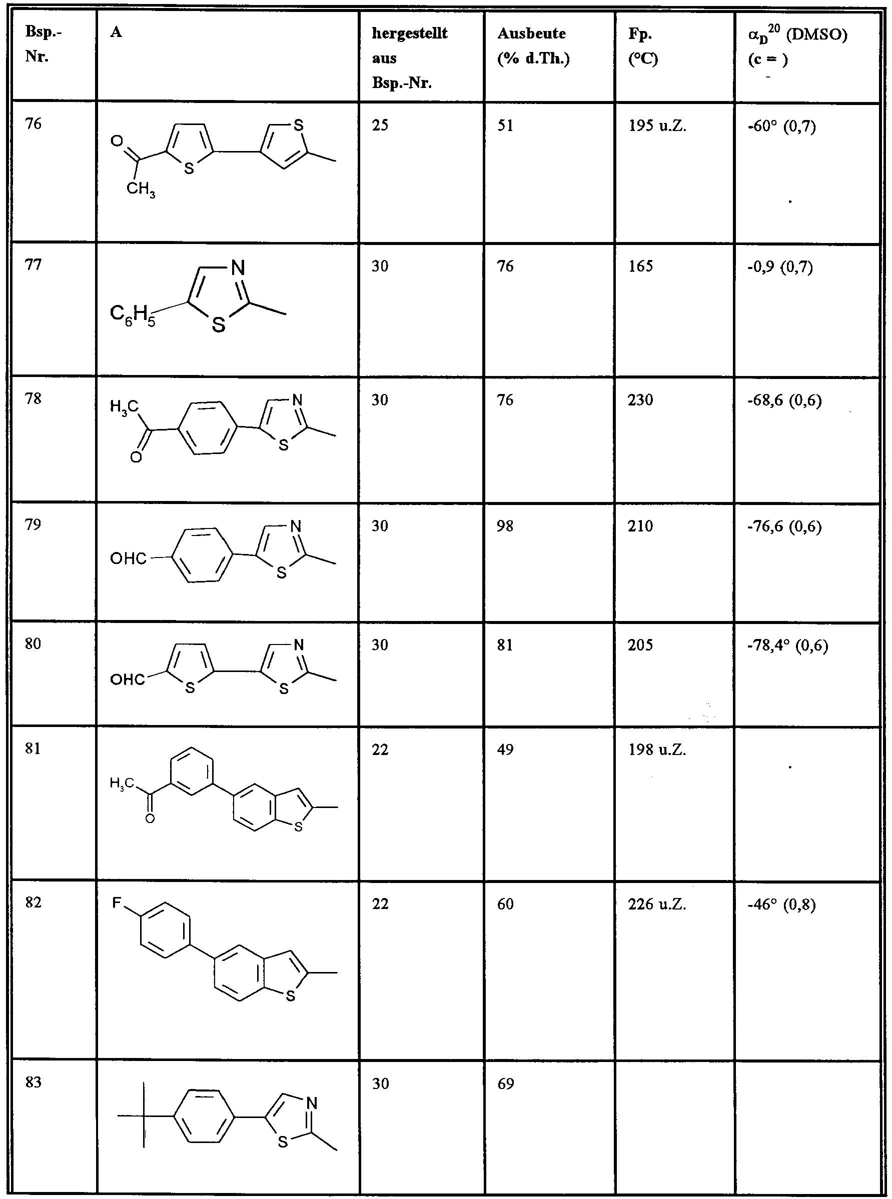

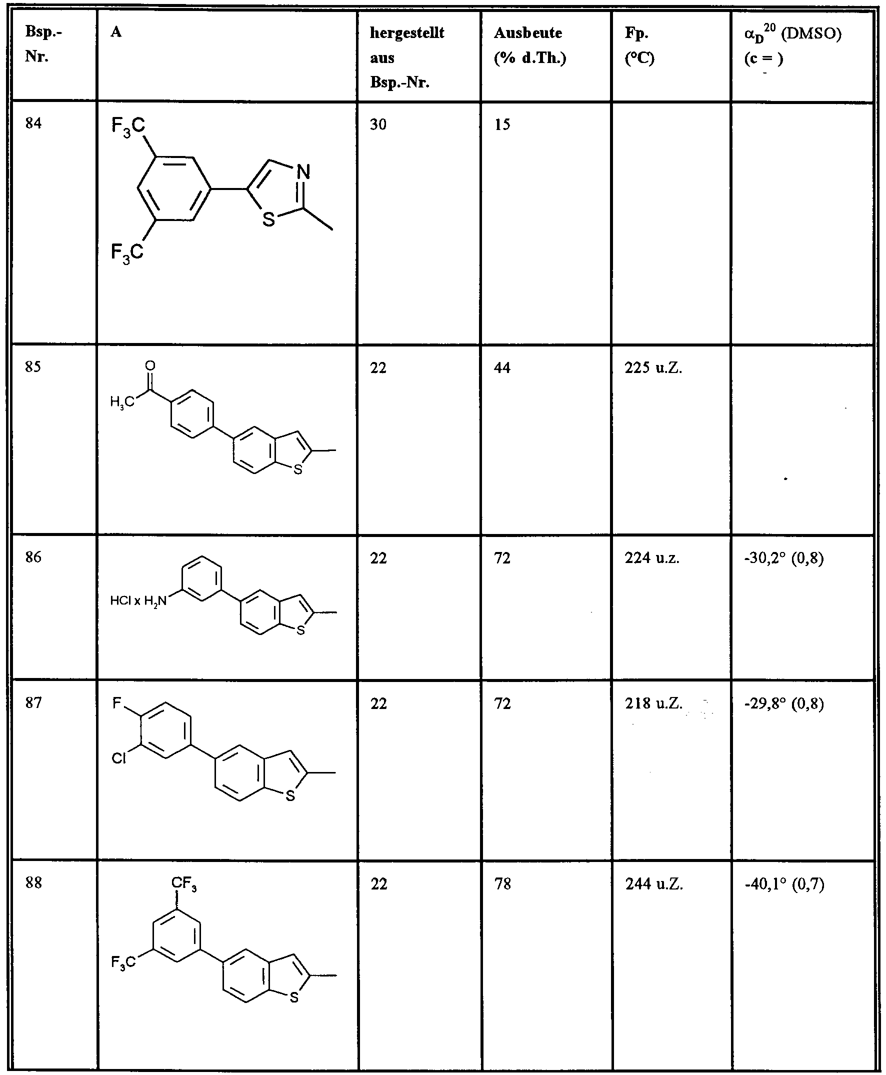

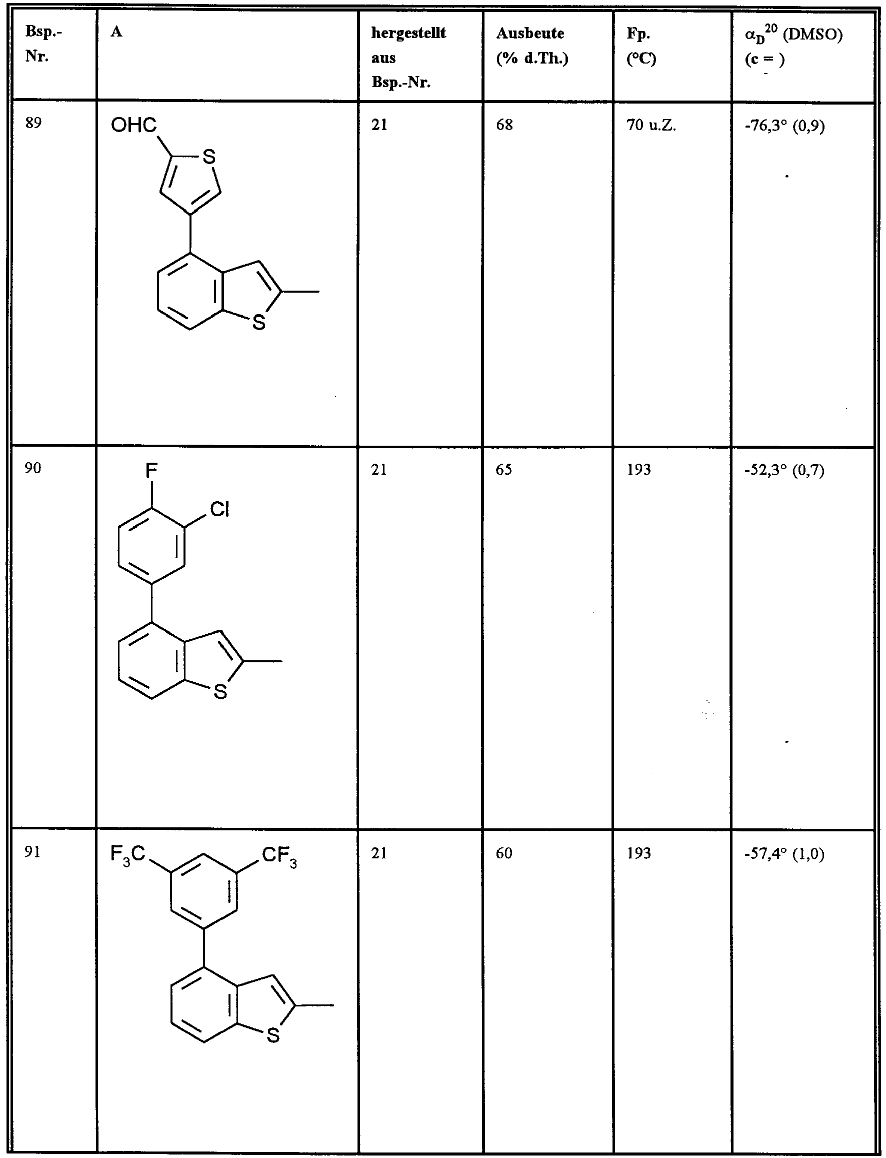

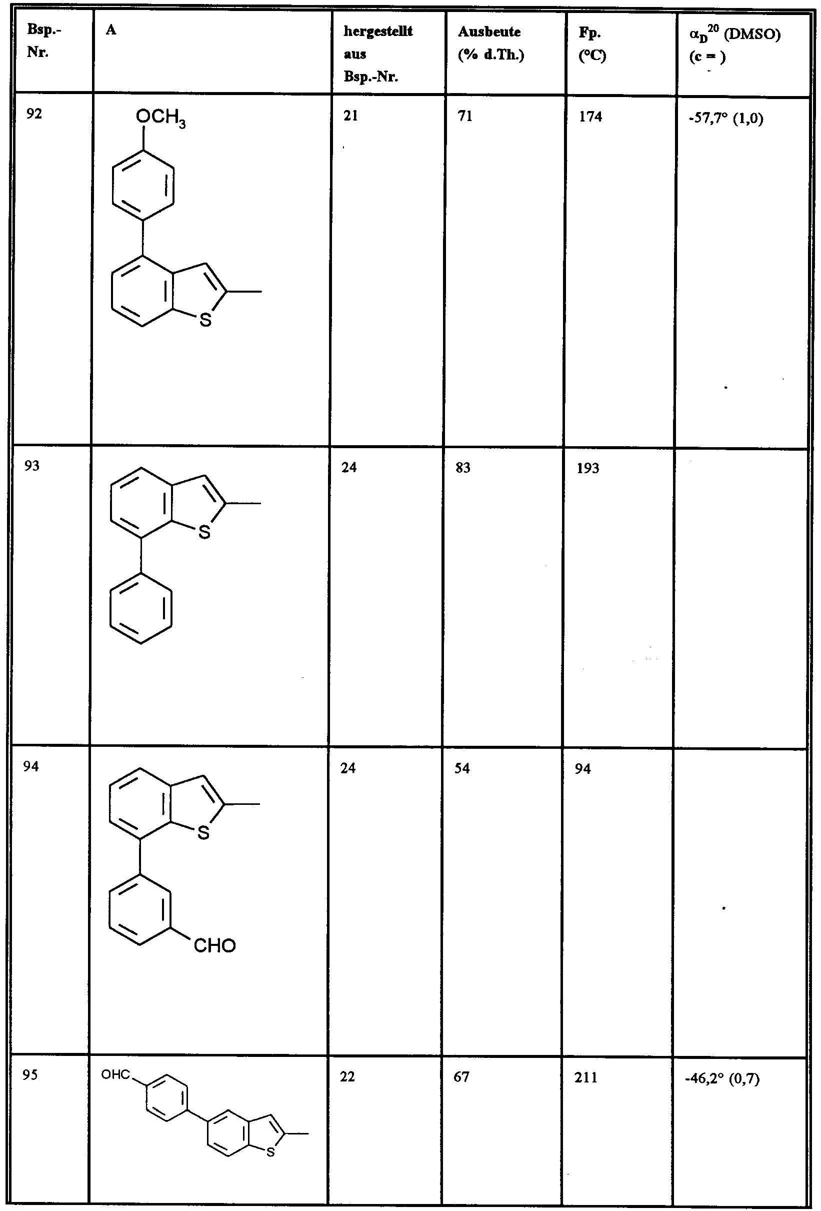

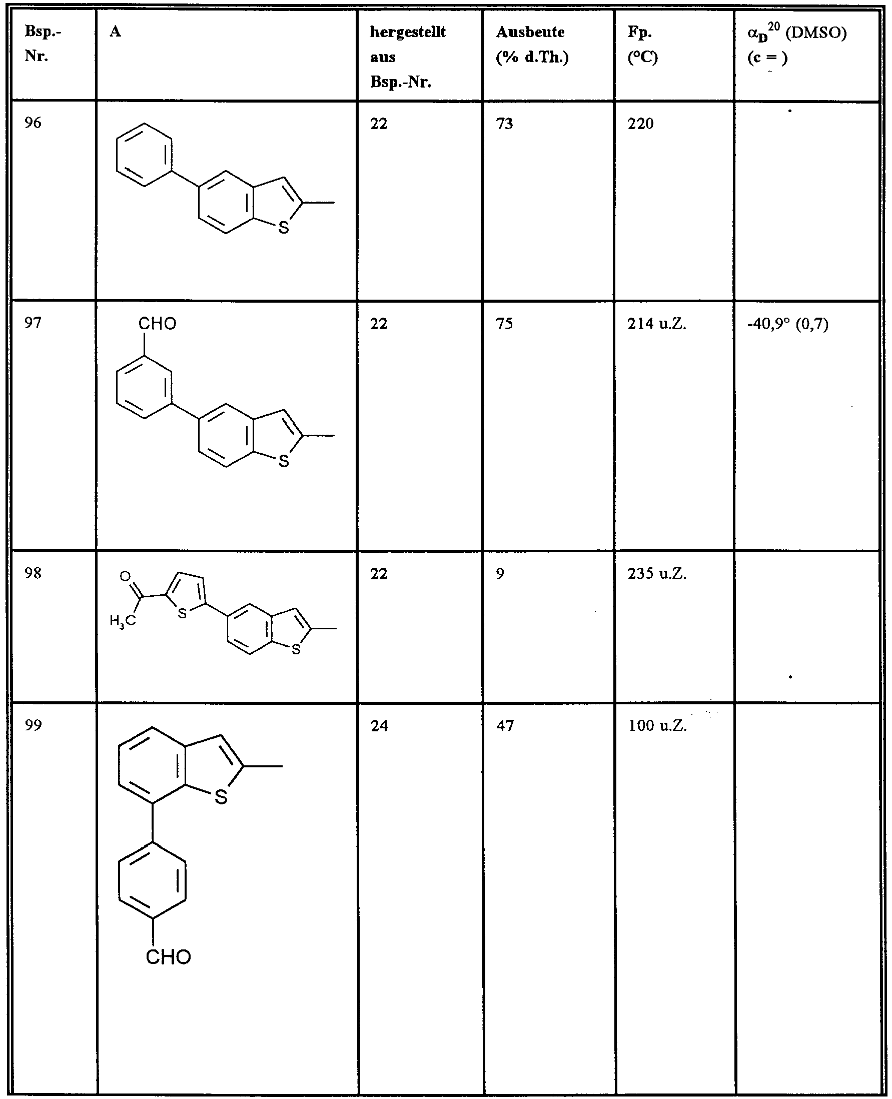

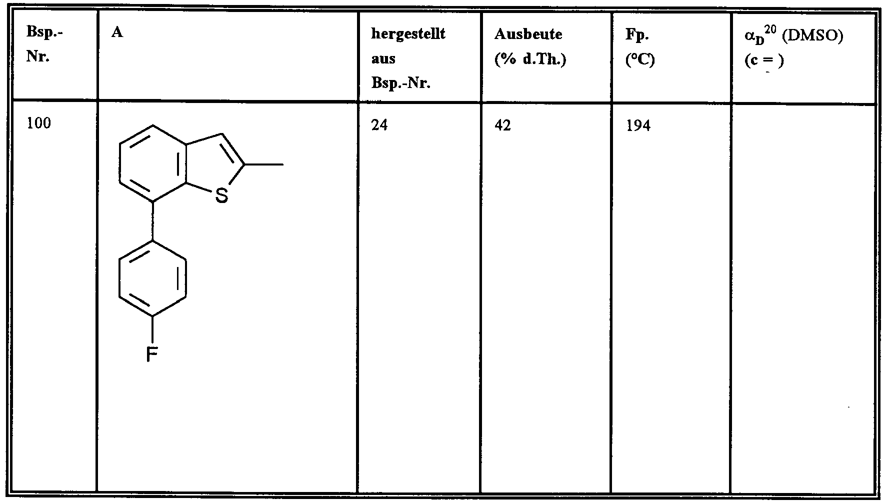

### Beispiel 101

### (5S)-3-(2-[5-(4-Hydroxymethyl)phenyl]thiophenyl)-5-acetylamino-methyl-oxazolidin-2-on

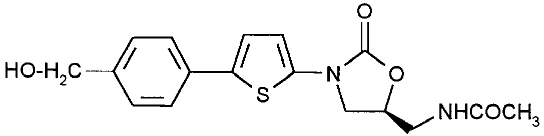

100 mg (0,29 mmol) der Verbindung aus Beispiel 44 werden in 3,2 ml Methanol gelöst, auf 0°C gekühlt und mit 6 mg (0,15 mmol) NaBH₄ versetzt; man hält 3 h bei 0°C, gibt 5 ml Wasser zu, rührt 1 h bei RT nach, saugt ab und trocknet.
Ausbeute: 69 mg (69%)
Smp.: 224°C n.Z.
MS (FAB): 346 [M⁺, 100%], 347 [M⁺+1, 80%]
¹H-NMR (D₆-DMSO, TMS): 8,28 (t, J= 7 Hz, 1 H); 7,55 (d, J = 9 Hz, 2H); 7,3 (d, J = 7 Hz, 2H); 7,28 (d, J = 5 HZ, 1 H); 6,51 (d, J = 5 Hz, 1 H); 5,19 (t, J = 6 Hz, 1 H); 4,78 - 4,92 (m, 1 H); 4,47 (d, J = 6 Hz, 1 H); 4,13 (t, J = 9 Hz, 1 H); 3,75 (dd, J = 9 Hz, J = 6 Hz, 1 H); 3,43 (t, J = 6 Hz, 2H); 1,89 (s, 3H).

In Analogie zur Vorschrift des Beispiels 101 werden die in der Tabelle 4 aufgeführten Verbindungen hergestellt:

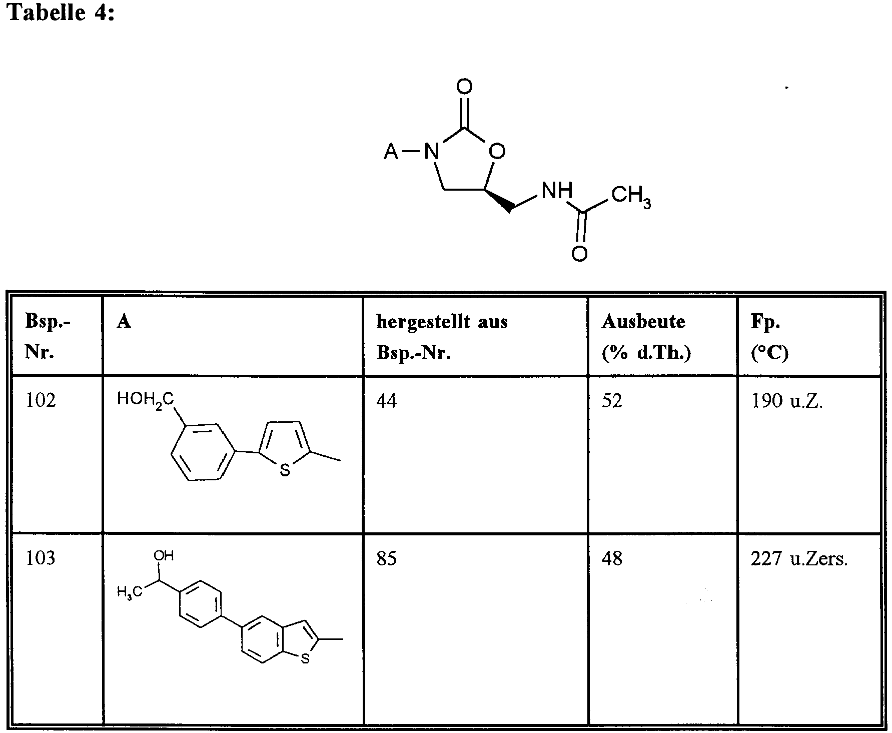

### Beispiel 104

### (5S)-3-(2-[5-(4-Carboxyphenyl)]thiophenyl)-5-acetyl-aminomethyl-oxazolidin-2-on

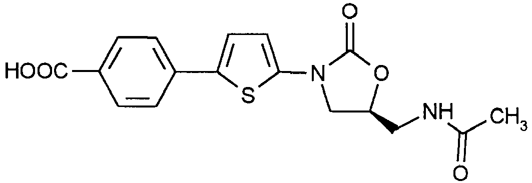

400 mg (1,16 mmol) der Verbindung aus Beispiel 44 werden in 40 ml Aceton und 5 ml Wasser gelöst und mit 211 mg (1,76 mmol) MgSO₄ und 190 mg (1,2 mmol) KMnO₄ versetzt. Man rührt über Nacht bei RT, gibt 5 ml Ethanol zu, kocht 10 min unter Rückfluß, gibt 10 ml gesättigte NaHCO₃-Lösung zu, saugt vom braunen Niederschlag ab, wäscht mit Wasser und mit Ether nach und trocknet im Vakuum bei 50°C.
Ausbeute: 114 mg (27%)
Smp.: 258°C u.Z.
¹H-NMR (D₆-DMSO, TMS): 12,95 (br, 1 H); 8,28 (t, J = 7 Hz, 1 H); 7,95 (d, J = 8 Hz, 2H); 7,72 (d, J = 8 Hz, 2H); 7,5 (d, J = 5 Hz, 1 H); 6,58 (d, J = 5 Hz, 1 H); 4,7 - 4,95 (m, 1 H); 4,17 (t, J= 9 Hz, 1 H); 3,77 (dd, J = 9 Hz, J = 6 Hz, 1 H); 3,43 (t, J = 6 Hz, 2H); 1,83 (s, 3H).

### Beispiel 105

### 4-(Benzo[b]furan-2-yl)-5-amidomethyloxazolidin-2-on

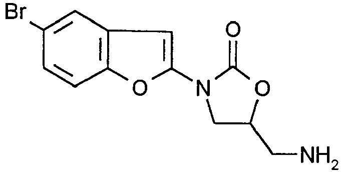

20 g (59,3 mmol) des entsprechenden Acids und 500 mg Platin(IV)oxid in 500 ml Methanol werden 3 h bei Raumtemperatur unter Wasserstoff (1 atm) gerührt. Es wird von Katalysator abfiltriert, das Lösungsmittel abgezogen und am Hochvakuum getrocknet.
Ausbeute: 19,0 g (quant.)
¹H-NMR (D₆-DMSO, TMS): 7,78 (d, J = 2Hz, 1 H), 7,50 (d, J = 8Hz, 1 H), 7,35 (dd, J = 8Hz, J = 2Hz, 1 H), 4,65-4,82 (m, 1 H), 4,22 (t, J = 9Hz, 1 H), 4,03 (dd, J = 9Hz, J = 5Hz, 1 H), 3,20-3,55 (bs, 2H), 2,70-2,95 (m,2H).

In Analogie zur Vorschrift des Beispiels 4 werden die in der Tabelle 5 aufgeführten Verbindungen hergestellt:

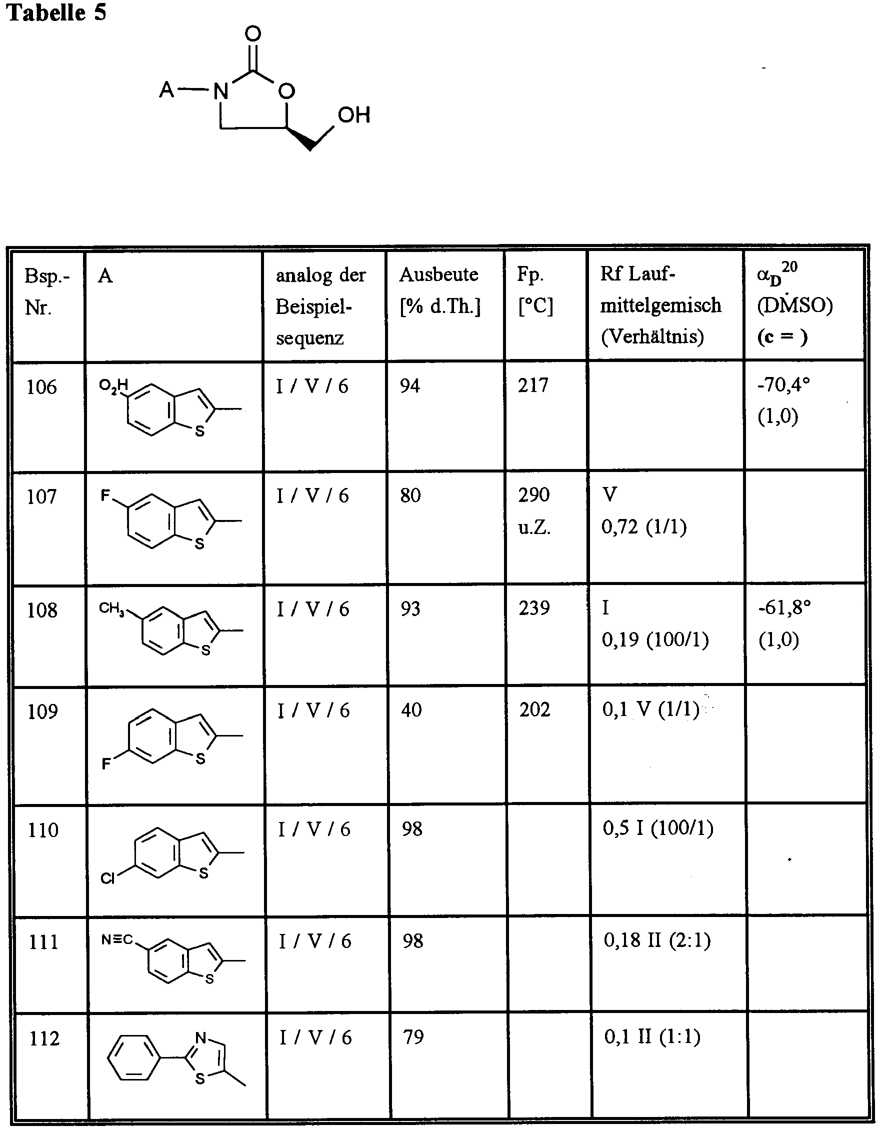

In Analogie zu den Vorschriften der Beispiele 18 bis 21 werden die in der Tabelle 6 aufgeführten Verbindungen hergestellt:

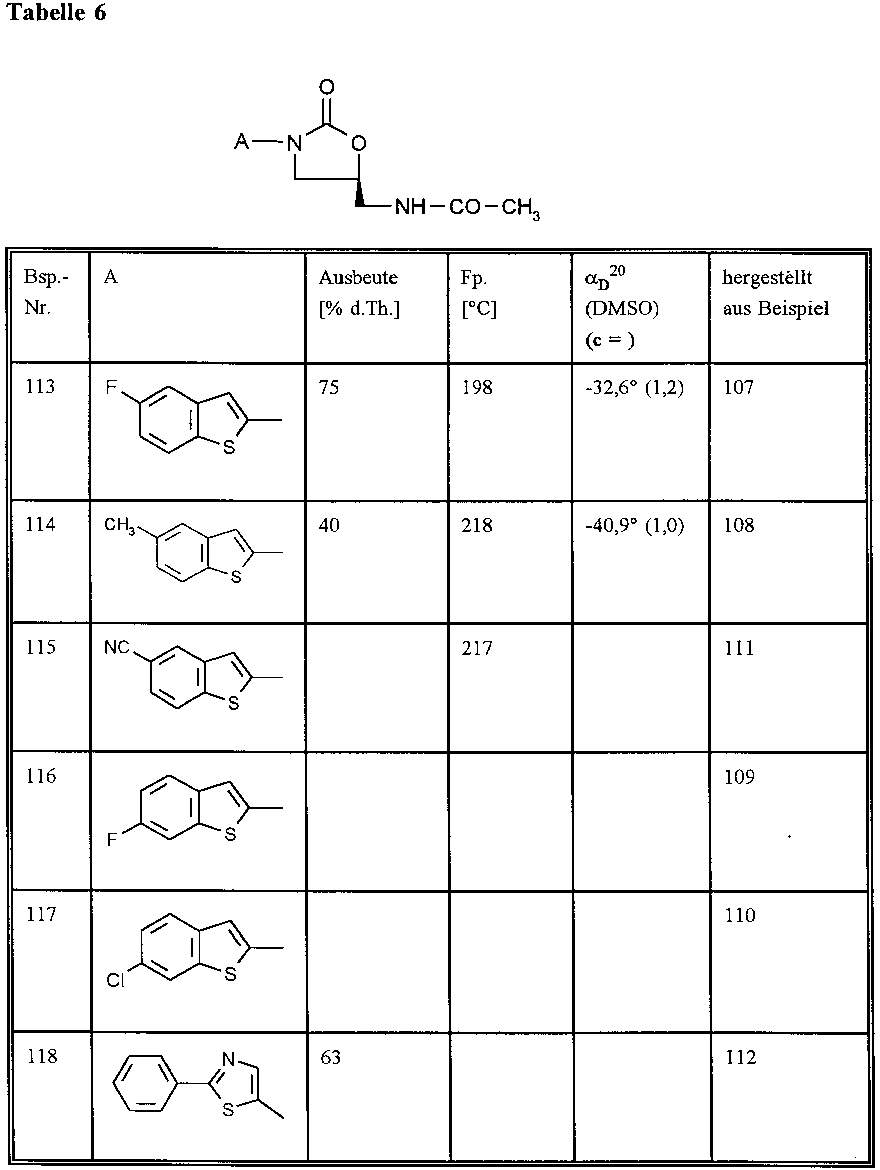

In Analogie zu den Vorschriften der Beispiele 39 bis 41 werden die in der Tabelle 7 aufgeführten Verbindungen hergestellt:

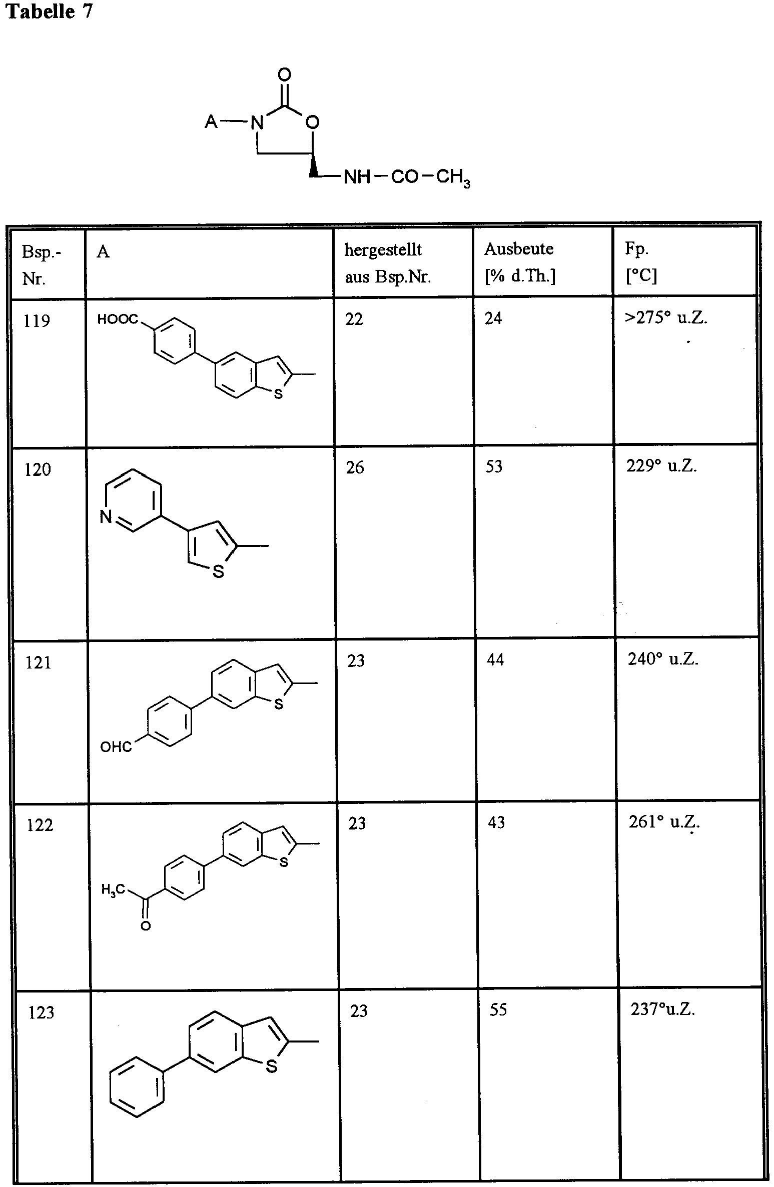

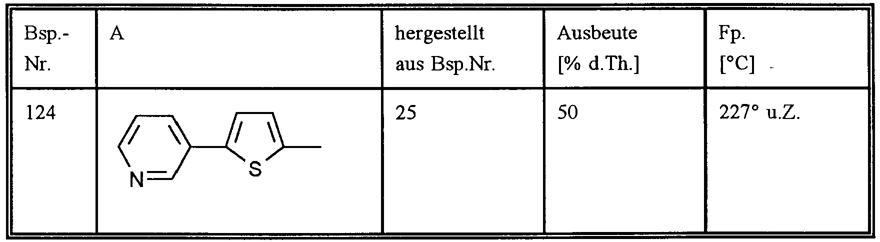

### Beispiel 125

### (5R)-3-[4-Carboxymethyl-benzo[b]-thiophenyl]-5-acetyl-aminomethyl-oxazolidin-2-on

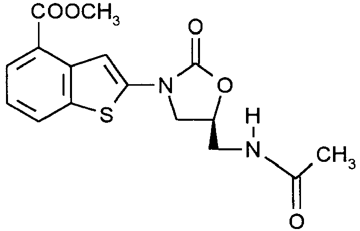

100 mg (0,27 mmol) der Verbindung aus Beispiel 21 werden in 4 ml Methanol und 2 ml THF gelöst, mit 2 ml Triethylamin und 31 mg (0,027 mmol) Pd(PPh₃)₂Cl₂ versetzt und 48 h am Rückfluß gekocht. Man engt ein und chromatographiert an Kieselgel mit Methylenchlorid/Methanol (100/0,5).
Ausbeute: 55 mg (58 %)
Rp: 0,36 (I; 100/5)
Smp: 193°C u.Z.
¹H-NMR (D₆-DMSO, TMS): δ = 8,28 (t, J = 6,5Hz, 1 H); 8,19 (d, J = 7,5Hz, 1 H); 8,02 (d, J = 7,5Hz, 1 H); 7,4 (s, 1 H); 7,36 (t, J = 7,5Hz, 1 H); 4,83-4,96 (m, 1 H); 4,30 (t, J = 9,5Hz, 1 H); 3,9 (dd, J = 9,5Hz, J = 6Hz, 1 H); 3,88 (s, 3H); 3,43-3,52 (m, 2H); 1,88 (s, 3H)
MSE: 348 (M⁺, 100 %)

In Analogie zur Vorschrift des Beispiels 125 werden die in der Tabelle 8 aufgeführten Verbindungen hergestellt:

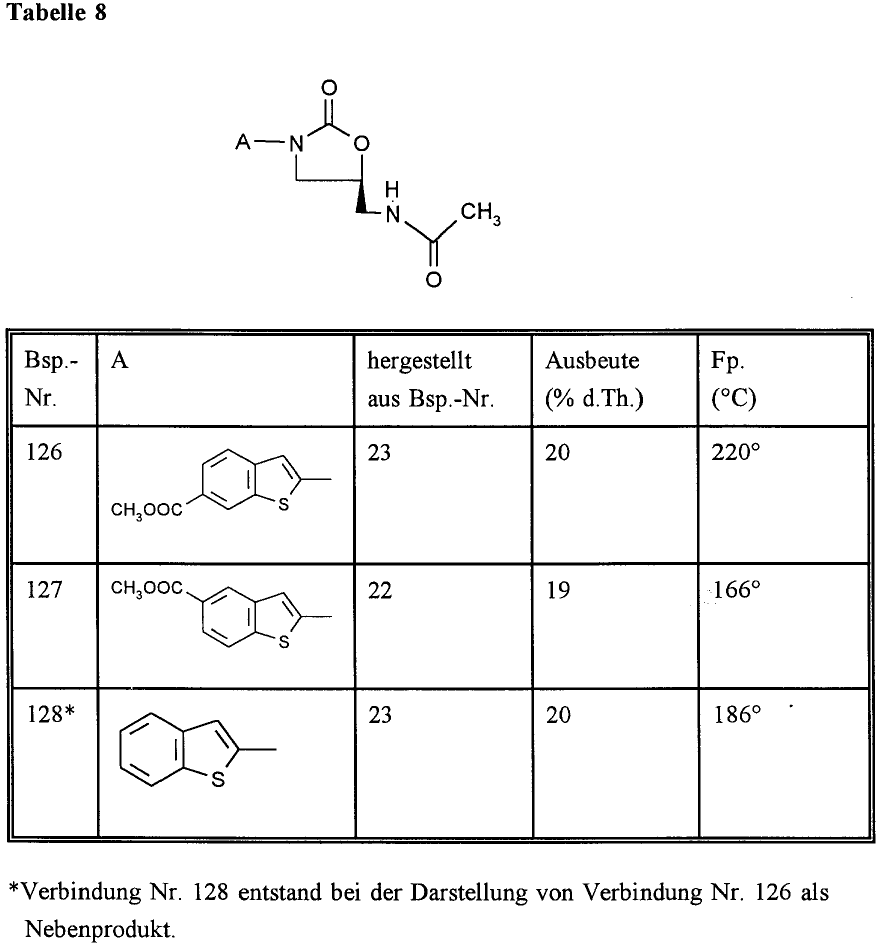

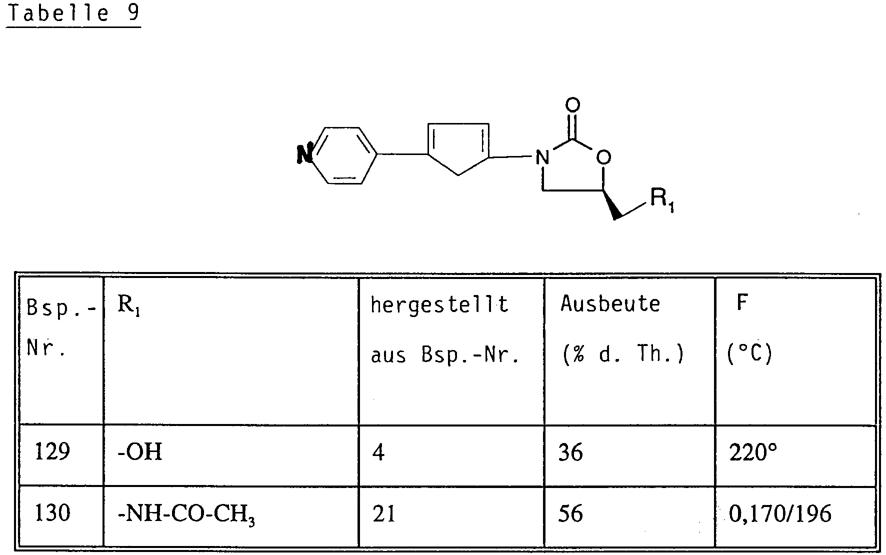

## Patentansprüche

1. Heteroaryl-oxazolidinone der allgemeinen Formel (I) 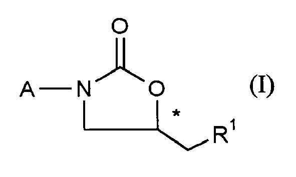 in welcher
R¹ für Azido, Hydroxy oder für eine Gruppe der Formel - OR², -O-SO₂R³ oder -NR⁴R⁵ steht,
worin
R² geradkettiges oder verzweigtes Acyl mit bis zu 8 Kohlenstoffatomen oder eine Hydroxyschutzgruppe bedeutet,
R³ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
R⁴ und R⁵ gleich oder verschieden sind und Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten,
oder
R⁴ oder R⁵ eine Gruppe der Formel -CO-R⁶ bedeutet,
worin
R⁶ Cycloalkyl mit 3 bis 6 Kohlenstoffatomen, geradkettiges oder verzweigtes Alkyl mit bis zu 8 Kohlenstoffatomen, Phenyl oder Wasserstoff bedeutet,
A für einen über ein Kohlenstoffatom direkt gebundenen 5-gliedrigen aromatischen Heterocyclus mit bis zu 3 Heteroatomen aus der Reihe S, N und/oder O steht, der zusätzlich einen annellierten Benzol-oder Naphthylring besitzen kann, wobei die Cyclen gegebenenfalls jeweils bis zu 3-fach gleich oder verschieden durch Carboxy, Halogen, Cyano, Mercapto, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR⁷R⁸ substituiert sein kann,
worin
R⁷ und R⁸ gleich oder verschieden sind und Wasserstoff, geradkettiges oder verzweigtes Alkyl mit bis zu 4 Kohlenstoffatomen oder Phenyl bedeuten,
oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,
und/oder
die Cyclen gegebenenfalls durch eine Gruppe der Formel -NR^{7'}R^{8'} substituiert sind, worin
R^{7'} und R^{8'} gleich oder verschieden sind und die oben angegebene Bedeutung von R⁷ und R⁸ haben und mit diesen gleich oder verschieden sind,
und/oder
die Cyclen gegebenenfalls durch (C₂-C₈)-Alkenylphenyl, Phenyl oder durch einen 5- oder 6-gliedrigen gesättigten oder ungesättigten Heterocyclus mit bis zu 3 Heteroatomen aus der Reihe S, N und/oder O substituiert sind, die ihrerseits gegebenenfalls durch eine Gruppe der Formel -CO-NR⁹R¹⁰, -NR¹¹ R¹², -NR¹³-S(O)₂-R¹⁴, R¹⁵R¹⁶N-SO₂-oder R¹⁷-S(O)ₐ- substituiert sind,
worin
a eine Zahl 0, 1 oder 2 bedeutet,
R⁹, R¹⁰, R¹³, R¹⁵ und R¹⁶ gleich oder verschieden sind und Wasserstoff, geradkettiges oder verzweigtes Alkyl mit bis zu 6 Kohlenstoffatomen oder Phenyl bedeuten,
R¹¹ und R¹² gleich oder verschieden sind und die oben angegebene Bedeutung von R⁷ und R⁸ haben und mit diesen gleich oder verschieden sind,
R¹⁴ und R¹⁷ gleich oder verschieden sind und die oben angegebene Bedeutung von R3 haben und mit dieser gleich oder verschieden sind,
und/oder ihrerseits gegebenenfalls bis zu 2-fach gleich oder verschieden durch Carboxy, Halogen, Cyano, Mercapto, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -NR¹⁸R¹⁹ substituiert sein kann,
worin
R¹⁸ und R¹⁹ die oben angegebene Bedeutung von R⁷ und R⁸ haben und mit diesen gleich oder verschieden sind,
und/oder
die Cyclen gegebenenfalls durch einen Rest der Formel 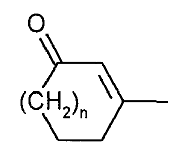 substituiert sind,
worin
n eine Zahl 0, 1 oder 2 bedeutet
und deren Salze und S-Oxide.

2. Heteroaryl-oxazolidinone nach Anspruch 1, bei denen
R¹ für Azido, Hydroxy oder für eine Gruppe der Formel -OR², -OSO₂R³ oder -NR⁴R⁵ steht,
worin
R² geradkettiges oder verzweigtes Acyl mit bis zu 6 Kohlenstoffatomen oder Benzyl bedeutet,
R³ geradkettiges oder verzweigtes Alkyl mit bis zu 3 Kohlenstoffatomen, Phenyl oder Toluolyl bedeutet,
R⁴ und R⁵ gleich oder verschieden sind und Cyclopropyl, Cycylopentyl, Cyclohexyl, Wasserstoff, Phenyl oder geradkettiges oder verzweigtes Alkyl mit bis zu 6 Kohlenstoffatomen, tert. Butoxycarbonyl oder Benzyloxycarbonyl bedeuten,
oder
R⁴ oder R⁵ eine Gruppe der Formel -CO-R⁶ bedeutet,
worin
R⁶ Cyclopropyl, Cyclobutyl, Cyclopentyl, Cyclohexyl oder geradkettiges oder verzweigtes Alkyl mit bis zu 6 Kohlenstoffatomen, Phenyl oder Wasserstoff bedeutet,
A für über ein Kohlenstoffatom direkt gebundenes Pyrrolyl, Imidazolyl, Furyl, Thienyl, Thiazolyl, Oxazolyl, Isothiazolyl, Isoxazolyl oder Furazanyl steht, oder für ebenfalls über ein Kohlenstoffatom des 5-gliedrigen Ringes direkt gebundenes Indolyl, Benzo[b]thienyl, Naphto[2,3-b]thienyl, Benzo[b]thiazolyl, Benzo[b]-imidazolyl oder Benzo[b]furanyl steht, wobei die Cyclen gegebenenfalls jeweils bis zu 3-fach gleich oder verschieden durch Carboxy, Fluor, Chlor, Brom, Jod, Cyano, Mercapto, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR⁷R⁸ gegebenenfalls substituiert sein kann,
worin
R⁷ und R⁸ gleich oder verschieden sind und Wasserstoff, geradkettiges oder verzweigtes Alkyl mit bis zu 3 Kohlenstoffatomen oder Phenyl bedeuten,
oder gemeinsam mit dem Stickstoffatom einen Morpholinyl-, Pyrrolidinyl-, Piperazinyl-oder Piperidylring bilden, die gegebenenfalls, auch über die freie N-Funktion, durch Methyl, Ethyl oder Acetyl substituiert sind,
und/oder die Cyclen gegebenenfalls durch eine Gruppe der Formel-NR^{7'}R^{8'} substituiert sind,
worin
R^{7'} und R^{8'} die oben angegebene Bedeutung von R⁷ und R⁸ haben und mit diesen gleich oder verschieden sind,
und/oder
die Cyclen gegebenenfalls durch (C₂-C₄)-Alkenylphenyl, Phenyl, Pyridyl oder Thienyl substituiert sind, die ihrerseits gegebenenfalls durch eine Gruppe der Formel - CO-NR⁹R¹⁰, -NR¹¹R¹², -NR13-SO₂-R¹⁴, R¹⁵R¹⁶N-SO₂- oder -R¹⁷-S(O)ₐ- substituiert sind,
worin
a eine Zahl 0, 1 oder 2 bedeutet,
R⁹, R¹⁰, R¹³, R¹⁵ und R¹⁶ gleich oder verschieden sind und Wasserstoff, geradkettiges oder verzweigtes Alkyl mit bis zu 4 Kohlenstoffatomen oder Phenyl bedeuten,
R¹¹ und R¹² gleich oder verschieden sind und die oben angegebene Bedeutung von R⁷ und R⁸ haben und mit diesen gleich oder verschieden sind,
R¹⁴ und R¹⁷ gleich oder verschieden sind und die oben angegebene Bedeutung von R3 haben und mit dieser gleich oder verschieden sind,
und/oder ihrerseits gegebenenfalls bis zu 2-fach gleich oder verschieden durch Carboxy, Fluor, Chlor, Brom, Jod, Cyano, Mercapto, Trifluormethyl, Formyl, Nitro, Phenyl, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -NR¹⁸R¹⁹ gegebenenfalls substituiert sein kann,
worin
R¹⁸ und R¹⁹ die oben angegebene Bedeutung von R⁷ und R⁸ haben und mit diesen gleich oder verschieden sind,
und/oder
die Cyclen gegebenenfalls durch einen Rest der Formel 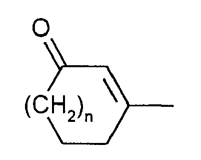 substituiert sind,
worin
n eine Zahl 0, 1 oder 2 bedeutet
und deren Salze und S-Oxide.

3. Heteroaryl-oxazolidinone nach Anspruch 1, bei denen
R¹ für Azido, Hydroxy oder für eine Gruppe der Formel - OR², -OSO₂R³ oder -NR⁴R⁵ steht,
worin
R² geradkettiges oder verzweigtes Acyl mit bis zu 6 Kohlenstoffatomen bedeutet,
R³ Methyl, Ethyl, Phenyl oder Toluolyl bedeutet,
R⁴ und R⁵ gleich oder verschieden sind und Cyclopropyl, Cyclopentyl, Cyclohexyl, Wasserstoff, Phenyl oder geradkettiges oder verzweigtes Alkyl mit bis zu 5 Kohlenstoffatomen bedeuten,
oder
R⁴ oder R⁵ eine Gruppe der Formel -CO-R⁶ bedeutet,
worin
R⁶ Cyclopropyl, Cyclopentyl, Cyclohexyl oder geradkettiges oder verzweigtes Alkyl mit bis zu 4 Kohlenstoffatomen, Wasserstoff oder Phenyl bedeutet,
A für über ein Kohlenstoffatom direkt gebundenes Pyrrolyl, Imidazolyl, Furyl, Thienyl, Thiazolyl, Isothiazolyl, Isoxazolyl, Furazanyl oder Oxazolyl steht, oder für ebenfalls über ein Kohlenstoffatom des 5-gliedrigen Ringes direkt gebundenes Indolyl, Benzo[b]thienyl, Benzo[b]imidazolyl, Benzo[b]furanyl oder Benzo[b]thiazolyl steht, wobei die Cyclen gegebenenfalls jeweils bis zu 2-fach gleich oder verschieden durch Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel - NR⁷R⁸ gegebenenfalls substituiert sein kann,
worin
R⁷ und R⁸ gleich oder verschieden sind und Wasserstoff oder Methyl bedeuten, oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind,
und/oder
gegebenenfalls durch eine Gruppe der Formel -NR^{7'}R^{8'} substituiert sind,
worin
R^{7'} und R^{8'} die oben angegebene Bedeutung von R⁷ und R⁸ haben und mit diesen gleich oder verschieden sind,
und/oder
die Cyclen gegebenfalls durch 2-Phenylvinyl, Phenyl, Pyridyl oder Thienyl substituiert sind, die ihrerseits gegebenenfalls durch eine Gruppe der Formel -CO-NR⁹R¹⁰ oder -NR¹¹ R¹² substituiert sind,
worin
R⁹ und R¹⁰ gleich oder verschieden sind und Wasserstoff oder Methyl bedeuten,
R¹¹ und R¹² gleich oder verschieden sind und die oben angegebene Bedeutung von R⁷ und R⁸ haben und mit dieser gleich oder verschieden sind,
und/oder ihrerseits gegebenenfalls bis zu 2-fach gleich oder verschieden durch Carboxy, Fluor, Chlor, Brom, Jod, Cyano, Formyl, Trifluormethyl, Nitro, Phenyl, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel - NR¹⁸R¹⁹ gegebenenfalls substituiert sein kann,
worin
R¹⁸ und R¹⁹ die oben angegebene Bedeutung von R⁷ und R⁸ haben und mit dieser gleich oder verschieden sind,
und/oder
die Cyclen gegebenenfalls durch einen Rest der Formel 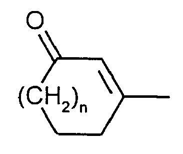 substituiert sind,
worin
n eine Zahl 0, 1 oder 2 bedeutet
und deren Salze und S-Oxide.

4. Verwendung der Heteroaryl-oxazolidinone nach den Ansprüchen 1 - 3 zur Herstellung von Arzneimitteln.

5. Arzneimittel, enthaltend Heteroaryl-oxazolidinone gemäß den Ansprüchen 1 - 3.
